# EUROPEAN PATENT SPECIFICATION

(11) **EP 1 373 494 B1**
(45) Date of publication and mention of the grant of the patent: **01.03.2006**
(21) Application number: 02718005.8
(22) Date of filing: 27.03.2002
(51) Int. Cl.: A61K 31/4015, A61K 31/402, A61K 31/4035, A61K 31/428, A61K 31/4706, A61K 31/473, A61K 31/513, A61K 31/52, A61P 35/00

(54) **SUCCINIMIDE AND MALEIMIDE DERIVATIVES AND THEIR USE AS TOPOISOMERASE II CATALYTIC INHIBITORS**
SUCCINIMID- UND MALEIMIDDERIVATE UND IHRE VERWENDUNG ALS KATALYTISCHE INHIBITOREN DER TOPOISOMERASE II
DERIVES DE SUCCINIMIDE ET DE MALEIMIDE ET LEUR UTILISATION COMME INHIBITEURS CATALYTIQUES DE LA TOPOISOMERASE II

(30) Priority: 29.03.2001 DK 200100522
(43) Date of publication of application: 02.01.2004
(73) Proprietor: Topo Target A/S, 2100 Copenhagen (DK)
(72) Inventor: JENSEN, Peter, Buhl, DK-3520 Farum (DK); SOKILDE, Birgitte, DK-3500 V rl se (DK); CARSTENSEN, Elisabeth, Vang, DK-3520 Farum (DK); LANGER, Seppo, W., DK-2820 Gentofte (DK); CREIGHTON, Andrew, London NW7 3QY (GB); SEHESTED, Maxvell, DK-2100 K benhavn (DK); JENSEN, Lars, Hallund, DK-2635 Ishoj (DK)
(74) Representative: Plougmann & Vingtoft A/S
(86) International application number: PCT/DK2002/000213
(87) International publication number: WO 2002/078679

(56) References cited:
- EP-A- 0 540 956
- WO-A-00/21927
- WO-A-00/54782
- WO-A-01/87307
- WO-A-97/34980
- DE-A- 19 838 506
- US-A1- 2002 035 090
- JENSEN L H ET AL: "Maleimide is a potent inhibitor of topoisomerase II in vitro and in vivo: A new mode of catalytic inhibition." MOLECULAR PHARMACOLOGY, vol. 61 , no. 5, 2002, pages 1235-1243, XP002902553
- SALMON L ET AL: "A general approach to the synthesis of polyamine linked-monoindolylmaleimides, a new series of trypanothione reductase inhibitors." CHEM. PHARM. BULL., vol. 46, no. 4, 1998, pages 707-710, XP002902554
- PRUDHOMME M: "Indolocarbazoles as anti-cancer agents." CURRENT PHARMACEUTICAL DESIGN, vol. 3, no. 3, 1997, pages 265-290, XP002902555
- HE M ET AL: "Design and synthesis of new leads for PKC bisubstrate inhibitors" BIOORGANIC & MEDICINAL CHEMISTRY LETTERS, vol. 4, no. 24, 1994, pages 2845-2850, XP002902556
- HASINOFF B B ET AL: "Mitindomide is a catalytic inhibitor of DNA topoisomerase II that acts at the bisdioxopiperazine binding site." MOLECULAR PHARMACOLOGY, vol. 52, 1997, pages 839-845, XP002902557
- ANIZON F ET AL: "Syntheses and biological activities (topoisomerase inhibition and antitumor and antimicrobial properties) of rebeccamycin analogues bearing modified sugar moieties and substituted on the imide nitrogen with a methyl group." JOURNAL OF MEDICINAL CHEMISTRY , vol. 40, no. 21, 1997, pages 3456-3465, XP002902558

## Description

### FIELD OF INVENTION

The present invention relates to maleimide and succinimide derivatives, including succinimide dimers linked by a tether, which act as topoisomerase II catalytic inhibitors. In particular, the present invention relates to the use of these compounds in the optimisation of anti-cancer treatment using currently used cytostatic agents which act as topoisomerase II poisons. The aims are to provide novel cytostatic agents for cancer treatment, to broaden the therapeutical index of classical anti-cancer agents, such as anthracyclines and epipodophyllotoxines, to reduce side effects caused by classical anti-cancer agents, such as extravasation.

### GENERAL BACKGROUND OF THE INVENTION

The topoisomerase II enzymes belong to a family of nuclear enzymes involved in the processing of DNA during the cell cycle. The essential nuclear enzyme topoisomerase II allows the separation of intertwined DNA strands by creating a transient double strand break in the DNA backbone thereby allowing the passage of another intact DNA double strand through the cleavage.

Topoisomerase II is the target of some of the most successful anti tumour agents used today, e.g. the epipodophyllotoxins etoposide (VP-16) and teniposide (VM-26) in the treatment of testicular and small cell lung cancer (1) and the anthracyclines.

The anthracyclines comprise a group of widely used cytotoxic compounds with activity in numerous malignant diseases. Daunorubicin and doxorubicin, the first anthracycline antibiotics to be discovered in the early 1960's, have a wide range of activity against malignant diseases - daunorubicin primarily in the field of haematological malignancies and doxorubicin against solid tumours. Epirubicin is a stereoisomer of doxorubicin with the same indications but slightly lesser potency and less cardiac toxicity than the parent drug. ldarubicin resembles daunorubicin. It is more lipophilic than the other anthracycline compounds and penetrates the blood-brain barrier more readily.

Drugs acting on topoisomerase II are divided into two main categories; topoisomerase II poisons and topoisomerase II catalytic inhibitors.

The topoisomerase II *poisons* shift the equilibrium of the catalytic cycle towards the cleavage of the DNA strands, thereby increasing the concentration of the transient protein-associated breaks in the genome (2). That is to say, they trap the cleavable complexes, which converts the essential topoisomerase II enzyme into a lethal one (3). Topoisomerase II *poisons* stabilise the cleavable complex by halting religation of the DNA in turn leading to the accumulation of the lethal double strand DNA breaks in the cell genome. There is solid evidence that etoposide, as well as a number of other clinically successful anti tumour agents such as daunorubicin and doxorubicin (4), are topoisomerase II poisons (5, 6). Although the precise cell killing mechanism is unknown, an obligatory step for the cytotoxicity of topoisomerase II poisons is related to an increase in cleavable complexes formation between DNA and topoisomerase II in treated cells (5).

The topoisomerase II *catalytic inhibitors* act through an entirely different mechanism. Rather than stimulating the cleavable complex, these drugs act by inhibiting the overall catalytic activity, of the topoisomerase II enzyme. *Catalytic inhibitors* act at different stages in the catalytic cycle than poisons do, acting on the topoisomerase II / DNA complex at stages in the catalytic cycle where DNA is not cleaved. This happens in at least two ways. (I) by Inhibiting the binding of topoisomerase II to DNA, thereby suppressing the interaction between the enzyme, the topoisomerase II targeting drug and the DNA. This is believed to be the case for chloroquine (7) and aclarubicin (8,9); (II) by locking topoisomerase II in its closed-clamp step after religation, which is the mode of action of the bisdioxopiperazines including ICRF-187 (10-14). By locking the enzyme in its closed damp formation, the bisdioxopiperazines hinder topoisomerase II poisons from exerting their cytotoxicity. The catalytic inhibitor of topoisomerase II, ICRF-187, abolishes both DNA breaks and cytotoxicity caused by the topoisomerase II poisons etoposide and daunorubicin (15).

The term "extravasation" is intended to relate to the escape of a chemotherapeutic drug from a vessel to the surrounding tissue, which may occur either by leakage of direct infiltration.

In cancer treatment, accidental extravasation is a feared complication, especially from drugs such as the anthracyclines, mitomycin, vincristine, and vinoorelbine, which are examples of vessicant drugs. Vessicant drugs cause tissue destruction upon infiltration. Extravasation is the unintended presence of a vessicant outside the vascular bed or vasculature.

The term "anti-extravasation agent" is intended to mean a compound which blocks or attenuates the local tissue toxicity caused by a vessicant.

Accidental extravasation has been estimated to occur in up to 6% of all patients receiving chemotherapy. Chemotherapeutic agents, such as the anthracyclines, are especially prone to cause severe tissue damage on extravasation. The tissue injury may not appear for several days or even weeks but when it appears it may continue to worsen for months, probably due to drug recycling into adjacent tissue. The local toxicity is characterised by acute pain, erythema, and swelling at the extravasation site and it often progresses to ulceration.

The present investigators have demonstrated, in animal studies, as well as on patients, that treatment with ICRF-187 protected against anthracycline extravasation injuries (19-21).

The bisdioxopiperazine ICRF-187 (dexrazoxane) is the water-soluble (+)-enantiomer of razoxane (ICRF-159). ICRF-187 is approved as a cardioprotective agent (Zinecard®, Cardioxane®) against anthracycline induced cardiotoxicity.

A hypothesis has been that ICRF-187, as an analogue of the cation binder EDTA, protects against free radical damage by binding to Fe⁺⁺ and thus concealing iron from oxygen. (16). However, the present investigators have recently demonstrated that cells with acquired resistance to ICRF-187 carry mutations in topoisomerase IIα (an isoform of topoisomerase II) which maps to different regions in topoisomerase II than those induced by topoisomerase II poisons such as daunorubicin and etoposide. The use of conditional expression of human topoisomerase II in yeast as well as characterization of purified topoisomerase II containing these mutations have confirmed that these mutations are functional (17,18). On this basis, it was concluded by the present investigators that ICRF-187 is a specific topoisomerase II agent.

Two models illustrate the use of catalytic inhibitors in pharmacologic regulation of topoisomerase II poisons (22). (I) A targeted high-dose treatment with etoposide based on physiological differences between normal (non-malignant) tissue and solid tumours may be achieved using the basic catalytic inhibitor chloroquine. At normal pH (in normal tissue), the chloroquine will (in its uncharged form) cross biomembranes thereby protecting the tissue from the poisonous attack. In cancer cells with weak acidic extracellular micro environment, the partly ionised catalytic inhibitor is no longer able to pass biomembranes, thus leaving the cancer cells exposed to the cytotoxic effect of the poison.

(II) Using ICRF-187, compartment directed high dose treatment with etoposide was obtained in the central nervous system (CNS) in a mouse model. In this case the protection of the peripheral tissue by the catalytic inhibitor is based on differences in lipophilicity between ICRF-187 and the topoisomerase II poison etoposide Studies concerning patients suffering from primary cancer outside the CNS show that about 25% of the patients develop brain metastasis. The risk of developing metastasis is dependent on the specific cancer form. The metastatic complications may be the patient's first symptom of cancer and may then produce serious neurological complications. Due to poor accessibility of the currently clinically used topoisomerase II poisons into the brain, the need for development of new catalytic inhibitors allowing dose escalation and thereby enhanced CNS effect is urgent.

The use of the catalytic inhibitor ICRF-187 in accidental extravasation caused by anthracydines is yet another example of reducing the side effects of topoisomerase II poisons hereby optimising the overall treatment of the patients: It is one object of the present invention to provide more compounds suitable for reducing side effects caused by chemotherapeutic agents and thereby optimising the overall treatment of cancer patients.

The use of super lethal doses of topoisomerase II poisons in combination with catalytic topoisomerase II inhibitors have proven an interesting strategy in the anticancer treatment. Selectivity can thus be obtained indirectly by the development of catalytic inhibitors capable of protecting specific targets thereby exerting the protective effect. Due to the non-optimal profile of the known catalytic inhibitors (toxidty, PK-properties etc) highly specific protection / cytotoxicity restriction is not obtained at present. Therefore there is an urgent need for the development of new catalytic inhibitors for use in the pharmalogical regulation of topoisomerase II poisons in anti cancer treatment.

Thus, the use of novel catalytic inhibitors of topoisomerase II included in the present invention will improve the anti-cancer treatment obtained with the classical topoisomerase II poisons resulting in a broader therapeutic index for these drugs by reducing the side effects (toxicity) or by enhancing the effect of the poison obtained by dose escalation.
There is also a urgent need for new compounds which are effective cytostatic or cytotoxic agents, themselves, in the treatment of cancer. A further aspect of the present invention is to provide for this need by providing compounds which are effective catalytic inhibitors of topoisomerase II and therefore effective chemotherapeutic agents in themselves. In this context novel compounds have been developed in order to obtain more effective catalytic inhibitors of topoisomerase II.

In testing the hypothesis that thiol-modification of DNA-bound topoisomerase II by quinones was involved in the stimulation of topoisomerase II-mediated DNA cleavage, Wang et al (28) tested a number of alkylating agents including quinones, N-ethylmaleimide (NEM), Disulfiram, and 2,2'-dithiobis-(5-nitropyridine) derivatives for their ability to stimulate topoisomerase II-mediated DNA cleavage. The results led Wang et al to postulate that these compounds act as topoisomerase II poisons by means of a Michael-addition to the enzyme. The present inventors have likewise examined the potential use of the thiol-reactive compounds maleimide and its N-substituted derivatives n-methyl-maleimide (NMM) and N-ethyl-maleimide (NEM) as topoisomerase II catalytic inhibitors. They have found that maleimide, NMM and NEM are potent catalytic inhibitors of purified human topoisomerase II α Maleimide and NEM are also able to antagonize etoposide induced topoisomerase II mediated DNA double strand cleavage in the test tube. Maleimide is further capable of protecting intact cells from etoposide induced DNA damage as well as from etoposide induced cytotoxicity. Finally, at MDR cell lines with reduced nuclear topoisomerase II content are fully sensitive to maleimide, indicating that it is not a topoisomerase II poison *in vivo.* These findings provide strong evidence that these compounds acts as topoisomerase II catalytic inhibitors and not as topoisomerase II poisons as reported in (28) The present inventers suggest that the observed antagonism *in vitro* and *in vivo* is caused by covalent modification of topoisomerase II cysteine residues reducing the amount of catalytically active enzyme sensitive to the action of topoisomerase II poisons, opening up for their use as novel topoisomerase II catalytic inhibitors. The inventors have further developed a new maleimide analogue TT0043 capable of antagonizing the cytotoxic effect of topoisomerase II poisons using cultured human cancer cells. The use of such novel thiol-reactive maleimide derivatives as catalytic inhibitors of topoisomerase II thus constitutes an important aspect of the present invention.

### SUMMARY OF THE INVENTION

In a first aspect, the invention relates to the use of a compound of formula I, quaternary ammonium salts thereof, or compositions comprising either entity, for the preparation of a medicament for the treatment or prevention of extravasation, 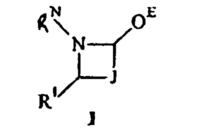
wherein -J- is 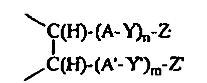
-O^{E} is a carbonyl equivalent such as selected from the group consisting of =O, =S; -OR²,--SR², dithiane and dioxolane;
R¹ is selected from the group consisting of -O^{E}, OR², N(R^{N})(R^{N}), S-R², NO₂, -CN, and halogen;
R^{N} is selected from the group consisting of hydrogen, optionally substituted C₁₋₆-alkyl, optionally substituted C₂₋₁₀-alkenyl, optionally substituted C₂₋₁₀-alkynyl, optionally substituted aryl, optionally substituted heteroaryl, optionally substituted heterocycle, optionally substituted C₃-C₇-cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³,--C(=O)-SR³, C(=O)-N(R³)(R³), C(C=S)-N(R³)(R³);
-A- and -A'- is selected from the group consisting of hydrogen, -C(R²)(R²)-, -C(=O)-,--N(R^{N})-, -O-, -S-, -P-, -P(O)-;
Y and Y' are each a biradical which may be absent or independently selected from one of the group consisting of optionally substituted C₁₋₆-alkyl, optionally substituted C(=O)-C₁₋₆₋alkyl, optionally substituted C₁₋₆-alkyl-C(=O), optionally substituted C₂₋₁₀-alkenyl, optionally substituted C₂₋₁₀-alkynyl, optionally substituted C₃₋₈-carbocycle and optionally substituted heterocycle;
Z and Z' are each a monoradical independently selected from the group consisting of optionally substituted aryl, optionally substituted heteroaryl, optionally substituted C₃₋₈₋carbocyle, optionally substituted heterocycle, H, OR^{Z}, N(R^{Z})(R³), S-R^{Z}, NO₂, -CN, and halogen;
wherein R² is selected from the group consisting of hydrogen, optionally substituted C₁₋₄ alkyl, optionally substituted C₂₋₅ alkenyl, optionally substituted C₂₋₆ alkynyl, optionally substituted aryl, optionally substituted heteroaryl, optionally substituted heterocycle, and optionally substituted C₃-C₇ cycloalkyl;
n is a whole number and m is a whole number, and wherein
R² and R³ are independently selected from the group consisting of hydrogen, halogen, hydroxy, optionally substituted C₁₋₆-alkyl, optionally substituted C₂₋₅ alkenyl, optionally substituted C₂₋₆ alkynyl, optionally substituted aryl, optionally substituted heteroaryl, optionally substituted heterocycle, and optionally substituted C₃-C₇ cycloalkyl.

Accordingly, the present invention relates to the use of a compound of formula I for the manufacture of a medicament for treating diseases and disorders for which inhibition or modulation of the topoisomerase II enzyme produces a physiologically beneficial response in said disease or disorder.

Compounds of the formula I, as topoisomerase inhibitors may be effective, in themselves, in the treatment of an array of forms of cancers. A further aspect of the present invention as is the use of compound of formula I for preparation of a medicament for the treatment of cancer.

Furthermore, compounds of the formula I, as topoisomerase inhibitors, may serve to broaden the therapeutic index of other chemotherapeutic agents. Compounds of the present invention may serve to reduce side effects assodated with other chemotherapeutic agents or by enhancing the effect of the chemotherapeutic agents. Thus, a further aspect of the present invention relates to the use of compounds of formula I in combination with at least one other chemotherapeutic agent for the manufacture of a medicament for the effective treatment of cancer.

Compounds of formula I are topoisomerase II catalytic inhibitors and, as such, may be used as pharmacological regulators of topoisomerase II which are exploited in a number of applications according to the present invention including the following:
1. To achieve dose escalation of classical topoisomerase II poisons such as etoposide (VP-16) as a mean of targeting the anti-tumour effect of such compounds to the central nervous system (CNS);
2. To target the cytotoxicity of known topoisomerase II poisons to acid microenvironments such as solid tumours;
3. To reduce the tissue destructive effect of known anticancer agents including topoisomerase II poisons in accidental extravasation occurring in the course of cancer treatment in general;
4. To act as anti-tumour agents in themselves my means of inhibiting essential topoisomerase II catalytic activity.

Thus, the compounds of formula I may be used for the manufacture of a medicament for preventing or treating tissue damage due to extravasation in an individual. The use of compounds of formula I for the manufacture of an anti-extravasation agent is an important aspect of the present invention.

### GENERAL DESCRIPTION OF THE INVENTION

As stated, the present invention relates to the use of a compound of formula I, quaternary ammonium salts thereof, or compositions comprising either entity, for the preparation of a medicament for the treatment or prevention of extravasation, 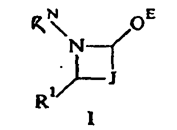
wherein -J- is 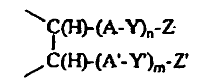
-O^{E} is a carbonyl equivalent such as selected from the group consisting of =O, =S; -OR²,--SR², dithiane and dioxolane;
R¹ is selected from the group consisting of -O^{E}, OR², N(R^{N})(R^{N}), S-R², NO₂, -CN, and halogen;
R^{N} is selected from the group consisting of hydrogen, optionally substituted C₁₋₆-alkyl, optionally substituted C₂₋₁₀-alkenyl, optionally substituted C₂₋₁₀-alkynyl, optionally substituted aryl, optionally substituted heteroaryl, optionally substituted heterocycle, optionally substituted C₃-C₇-cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³,--C(=O)-SR³, C(=O)-N(R³)(R³), C(C=S)-N(R³)(R³);
-A- and -A'- is selected from the group consisting of hydrogen, -C(R²)(R²)-, -C(=O)-,--N(R^{N})-, -O-, -S-, -P-, -P(O)-;
Y and Y' are each a biradical which may be absent or independently selected from one of the group consisting of optionally substituted C₁₋₆-alkyl, optionally substituted C(=O)-C₁₋₆₋alkyl, optionally substituted C₁₋₆-alkyl-C(=O), optionally substituted C₂₋₁₀-alkenyl, optionally substituted C₂₋₁₀-alkynyl, optionally substituted C₃₋₈-carbocycle and optionally substituted heterocycle;
Z and Z' are each a monoradical independently selected from the group consisting of optionally substituted aryl, optionally substituted heteroaryl, optionally substituted C₃₋₈₋carbocyle, optionally substituted heterocycle, H, OR^{Z}, N(R^{Z})(R³), S-R^{Z}, NO₂, -CN, and halogen;
wherein R^{Z} is selected from the group consisting of hydrogen, optionally substituted C₁₋₄ alkyl, optionally substituted C₂₋₅ alkenyl, optionally substituted C₂₋₆ alkynyl, optionally substituted aryl, optionally substituted heteroaryl, optionally substituted heterocycle, and optionally substituted C₃-C₇ cycloalkyl;
n is a whole number and m is a whole number, and wherein
R² and R³ are independently selected from the group consisting of hydrogen, halogen, hydroxy, optionally substituted C₁₋₆-alkyl, optionally substituted C₂₋₅alkenyl, optionally substituted C₂₋₆ alkynyl, optionally substituted aryl, optionally substituted heteroaryl, optionally substituted heterocycle, and optionally substituted C₃-C₇ cycloalkyl.

In the present context the term "C₁₋₆-alkyl" is intended to mean a linear or branched saturated hydrocarbon chain wherein the longest chains has from one to six carbon atoms, such as methyl, ethyl, n-propyl, isopropyl, n-butyl, isobutyl, sec-butyl, tert-butyl, pentyl, isopentyl, neopentyl and hexyl. A branched hydrocarbon chain is intended to mean a C₁₋₆₋alkyl substituted at any carbon with a hydrocarbon chain.

In the present context the term "C₂₋₁₀-alkenyl" is intended to mean a linear or branched hydrocarbon group having from two to eight carbon atoms and containing one or more double bonds. Examples of C₂₋₈-alkenyl groups include allyl, homo-allyl, vinyl, crotyl, butenyl, pentenyl, hexenyl, heptenyl and octenyl. Examples of C₂₋₁₀-alkenyl groups with more than one double bond include butadienyl, pentadienyl, hexadienyl, heptadienyl, hexatrienyl, heptatrienyl and octatrienyl groups as well as branched forms of these. The position of the unsaturation (the double bond) may be at any position along the carbon chain and the term is intended to include alkylidene groups.

In the present context the term "C₂₋₁₀-alkynyl" is intended to mean linear or branched hydrocarbon groups containing from two to ten carbon atoms and containing one or more triple bonds. Examples of C₂₋₁₀-alkynyl groups include acetylene, propynyl, butynyl, pentynyl, hexynyl, heptynyl and octynyl groups as well as branched forms of these. The position of unsaturation (the triple bond) may be at any position along the carbon chain the term is intended to include alkylidyne groups. More than one bond may be unsaturated such that the "C₂₋₁₀-alkynyl" is a di-ynes or enedi-ynes as it is known to the person skilled in the art.

In the present context the term "C₃₋₈-cycloalkyl" is intended to cover three-, four-, five-, six- seven-, and eight-membered rings comprising carbon atoms only whereas the term "heterocyclyl" is intended to mean three-, four-, five-, six- seven-, and eight-membered rings wherein carbon atoms together with from 1 to 3 heteroatoms constitute said ring. The heteroatoms are independently selected from oxygen, sulphur, and nitrogen.

C₃₋₈-cycloalkyl and heterocyclyl rings may optionally contain one or more unsaturated bonds situated, however, in such a way that an aromatic a-electron system does not arise.

Examples of preferred "C₃₋₈-cycloalkyl" are the carbocycles cyclopropane, cyclobutane, cyclopentane, cyclopentene, cyclopentadiene, cyclohexane, cyclohexene, 1,3-cyclohexadiene, 1,4-cyclohexadiene, cycloheptane, cycloheptene, 1,2-cycloheptadiene, 1,3-cycloheptadiene, 1,4-cycloheptadiene and 1,3,5 cycloheptatriene.

Examples of "heterocycles" are the heterocycles 2*H*-thipyran, 3*H*-thipyran, 4*H*-thipyran, tetrahydrothiopyran, 2*H*-pyran, 4*H*-pyran, tetrahydropyran, piperidine, 1,2-dithlin, 1,2-dithiane, 1,3-dithlin, 1,3-dithiane, 1,4-dithiln, 1,4-dithiane, 1,2-dioxin, 1,2-dioxane, 1,3-dioxin, 1,3-dioxane, 1,4-dioxin, 1,4-dioxane, piperazine, 1,2-oxathiin, 1,2-oxathiane, 4*H-*1,3-oxathlin, 1,3-oxathiane, 1,4-oxathiln, 1,4-oxathiane, 2*H*-1,2-thiazine, tetrahydro-1,2-thiazine, 2*H*-1,3-thiazine, 4*H*-1,3-thiazine, 5,6-dihydro-4*H*-thiazine, 4*H*-1,4-thiazine, tetrahydro-1,4-thiazine, 2*H*-1,2-oxazine, 4*H*-1,2-oxazine, 6*H*-1,2-oxazine, 2*H*1,3-oxazine, 4*H*-1,3-oxazine, 4*H*-1,4-oxazine, maleimide, succinimide, imidazole, pyrazole, pyrrole, oxazole, furazan, barbituric acid, thiobarbituric add, dioxopiperazine, isoxazole, hydantoin, dihydrouracil, morpholine, trioxane, 4*H*-1,2,3-trithiin, 1,2,3-trithiane, 1,3,5-trithiane, hexahydro-1,3,5-triazine, tetrahydrothiophene, tetrahydrofuran, pyrroline, pyrrolidine, pyrrolidone, pyrrolidione, pyrazoline, pyrazolidine, imidazoline, imidazolidine, 1,2-dioxole, 1,2-dioxolane, 1,3-dioxole, 1,3-dioxolane, 3*H*-1,2-dithiole, 1,2-dithiolane, 1,3-dithiole, 1,3-dithiolane, isoxazoline, isoxarolidine, oxazoline, oxazolidine, thiazoline, thiozolidine, 3*H*-1,2-oxathiole, 1,2-oxathiolane, 5*H*-1,2-oxathiole, 1,3-oxathiole, 1,3-oxathiolane, 1,2,3-trithiole, 1,2,3-trithiolane, 1,2,4-trithiolane, 1,2,3-trioxole, 1,2,3-trioxolane. 1,2,4-trioxolane, 1,2,3-triazoline and 1,2,3-triazolidine.

In the present context the term "aryl" is intended to mean a carbocyclic aromatic ring or ring system. Moreover, the term "aryl" includes fused ring systems wherein at least two aryl rings, or at least one aryl and at least one C₃₋₈-cycloalkyl, or at least one aryl and at least one heterocyclyl, share at least one carbon atom, such as in a spiro sytem or at least chemical bond, such as in a fused system. Examples of "aryl" rings include optionally substituted phenyl, naphthalenyl, phenanthrenyl, anthracenyl, acenaphthylenyl, tetralinyl, fluorenyl, indenyl, indolyl, cournaranyl, coumarinyl, chromanyl, isochromanyl, and azulenyl. A preferred aryl group is phenyl.

In the present context, the term "heteroaryl" is intended to mean an aryl group where one or more carbon atoms in an aromatic ring have been replaced with one or more heteroatoms selected from the group comprising nitrogen, sulphur, phosphorous and oxygen. Furthermore, in the present context, the term "heteroaryl" comprises fused ring systems wherein at least one aryl ring and at least one heteroaryl ring, at least two heteroaryls, at least one heteroaryl and at least one heterocyclyl, or at least one heteroaryl and at least one C₃₋₈-cycloalkyl share at least one chemical bond, such as two chemical bonds. Examples of a heteroaryl may be selected from the group comprising furanyl, thiophenyl, pyrrolyl, phenoxazonyl, oxazolyl, thiazolyl, isothiazolyl, imidazolyl, pyrazolyl, isoxazolyl, imidazolyl isothiazolyl, oxadiazolyl, furazanyl, triazolyl, thiadiazolyl, piperidinyl, pyridinyl, pyridazinyl, pyrimidinyl, pyrazinyl, pyrazolyl and triazinyl, isoindolyl, indolinyl, benzofuranyl, benzothiophenyl, benzopyrazolyl, indazolyl, benzimidazolyl, benzthiazolyl, purinyl, quinolizinyl, quinolinyl, isoquinolinyl, cinnolinyl, phthalazinyl, quinazolinyl, quinoxalinyl, naphthyridinyl, pteridinylthienofuranyl, carbazolyl, acridinyl, phenazinyl, phenothiazinyl, phenoxazinyl and thianthrenyl.

When used herein the term "C₁₋₆-alkoxy" is intended to mean C₁₋₆-alkyl-oxy such as methoxy, ethoxy, n-propoxy, isopropoxy, n-butoxy, isobutoxy, sec-butoxy, tert-butoxy, pentoxy, isopentoxy, neopentoxy and hexoxy

The term "halogen" Includes fluorine, chlorine, bromine and iodine.

In the present context, i.e. in connection with the terms "aryl", "heteroaryl", "C₃₋₈₋cycloalkyl", "heterocyclyl", "C₁₋₆-alkyl", "C₁₋₆-alkoxy", "C₂₋₈-alkenyl", and "C₂₋₈-alkynyl", the term "optionally substituted" is intended to mean that the group in question may be substituted one or several times, such as 1 to 5 times, preferably 1 to 3 times, most preferably 1 to 2 times, with one or more groups selected from C₁₋₆-alkyl, C₁₋₆-alkoxyl, oxo (which may be represented in the tautomeric enol form), carboxyl, amino, hydroxyl (which when present in an enol system may be represented in the tautomeric keto form), nitro, sulphono, sulphanyl, sulfoxide, C₁₋₆-carboxyl, C₁₋₆-alkoxycarbonyl, C₁₋₆-alkylcarbonyl, formyl, aryl, aryloxy, aryloxycarbonyl, arylcarbonyl, heteroaryl, amino, mono- and di(C₁₋₆₋alkyl)amino; carbamoyl, mono- and di(C₁₋₆-alkyl)aminocarbonyl, amino-C₁₋₆-alkylaminocarbonyl, mono- and di(C₁₋₆-alkyl)amino-C₁₋₆-alkyl-aminocarbonyl, C₁₋₆₋alkylcarbonylamino, cyano, guanidino, carbamido, C₁₋₆-alkanoyloxy, C₁₋₆-alkylsulphonyloxy, dihalogen-C₁₋₆-alkyl, trihalogen-C₁₋₆-alkyl, halogen, where aryl and heteroaryl representing substituents may be substituted 1-3 times with C₁₋₆-alkyl, C₁₋₆-alkoxy, sulfoxide, nitro, cyano, hydroxy, amino or halogen. In general, the above substituents may be susceptible to further optional substitution.

The term "salts" is intended to mean pharmaceutically acceptable acid addition salts obtainable by treating the base form of a functional group, such as an amine, with appropriate acids such as inorganic acids, for example hydrohalic acids; typically hydrochloric, hydrobromic, hydrofluoric, or hydroiodic acid; sulfuric acid; nitric acid; phosphoric acid and the like; or organic acids, for example acetic, propionic, hydroacetic, 2-hydroxypropanoic acid, 2-oxopropanoic acid, ethandioic, propanedioic, butanedioic, (Z)-2-butenedioic, (E)-butenedioic, 2-hydroxybutanedioic, 2,3-dihydroxybutanedioic, 2-hydroxy-1,2,3-propanetricarboxylic, methanesulfonic, ethanesulfonic, 2-hydroxyethanesulfonic acid, benzenesulfonic, 4-methylbenzenesulfonic acid, cyclohexanesulfamic, 2-hydoxybenzoic, fumaric acid, 4-amino-2-hydroxybenzoic, and other acids known to the skilled practitioner.

The term "carbonyl equivalent" is intended to mean derivatives of the carbonyl functional group as known to the person skilled in the art and are intended to include alkylated tautomers thereof, protected forms of the functional group and reduced-protected forms of the functional group.

The present compounds of formula I were surprisingly found to be catalytic inhibitors of topoisomerase II. Compounds of formula I have never been associated with topoisomerase II, nor for cancer treatment. Compounds of formula I can be loosely described as succinimide derivatives (compounds of formula D), whereas compounds of formula M are maleimide derivatives, both differing notably from the bisdioxopiperazine ICRF-187 (dexrazoxane), which is a highly specific topoisomerase II catalytic inhibitor. Compounds of formula I may be the compounds of formula M wherein at least one of R⁶ and R⁷ are independently selected from a group consisting of hydrogen, halogen, hydroxy, primary, secondary or tertiary amine, optionally substituted 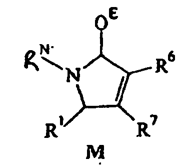 C₁₋₆-alkyl, optionally substituted C₂₋₅-alkenyl, optionally substituted C₂₋₆-alkynyl, optionally substituted aryl, optionally substituted heteroaryl, optionally substituted heterocycle, and optionally substituted C₃-C₇ cycloalkyl; and the other of R⁶ and R⁷ is A-Y-Z, as defined supra.

The present investigators have surprisingly found that the compounds of formula M, such as maleimide, NNM and NEM, function as topoisomerase catalytic inhibitors in vitro as well as in intact cells. Contrary to the Wang et al's teaching that NEM increases the level of DNA cleavage with purified topoisomerase II in vitro, the present investigators have found that NEM as well as maleimide does not stimulate DNA cleavage in vitro. On the contrary, the present inventors find that maleimide and NEM are both capable of antagonising VP-16 induced topoisomerase II mediated plasmid DNA cleavage in vitro. Additionally, the present inventors have found that the compounds of formula M only induce modest levels of DNA damage in NYH cells, at concentrations up to 250 uM while expensive DNA damage is provided by 3 uM etoposide. Also, in alkaline elution assays, the compound of formula M, maleimide, was found to antagonize DNA damage induced by etoposide, daunorubicin or doxorubicin. Thus, the present inventors find that compounds of formula M behave not as topoisomerase II poisons but rather as topoismerase II inhibitors.

This was confirmed in a clonogenic assay where maleimide, N-methyl-maleimide (NMM), N-ethyl-maleimlde (NEM) as well as TT0043 effectively blocked the cytotoxicity of etoposide and daunorubicin, providing furtrher evidence that compounds of formula M are catalytic inhibitors of toposiomerase II. Further corroboration of catalytic inhibitory activity of compounds of formula M was found in clonogenic assays on human lung cancer cell lines H69/dau and H69/VP because no resistance to compounds of formula M was found in these cell lines which are selected to be daunorubicin and, etopside (VP-16) resistant respectively. These lines exhibit cross-resistance to all known topoisomerase II poisons This finding strengthens the notion that compounds of formula M are not topoisomerase II poisons but catalytic topoisomerase II inhibitors.

Accordingly, in preferred embodiments of compounds of formula M, R¹ is preferably selected from the group consisting of =O^{E} and OR⁴, most preferably wherein R¹ is the carbonyl equivalent =O^{E}. As is known to the person skilled in the art, however, carbonyl groups (C=O) may be protected so as to dampen their reactivity. Thus, the carbonyl group or groups of compound M may be protected in a manner known to the person skilled in the art, such as its dioxolane or dithiane. Similarly, the carbonyl equivalent may be a thiocarbonyl or protected forms thereof. Alternatively, tautomers of the carbonyl or thiocarbonyl may be prepared and protected with, for instance, an alkyl chain. In a preferred embodiment of compounds of formula M, R¹ is =O.

In a further interesting embodiment of the present invention, the imide nitrogen is derivatized so as to render the compounds of formula M suitable pro-drugs for administration. In suitable embodiment of the compounds, R^{N} is selected from the group consisting of hydrogen, C₁₋₆-alkyl, optionally substituted aryl, optionally substituted heteroaryl, optionally substituted CH₂-aryl, CH₂-N(R⁴)(R⁴), CH₂-OR⁴, CH₂-SR⁴, CH₂-O-C(=O)R⁴, CH₂-O-C(=S)R⁴. In particularly interesting embodiments of compounds of formula M, R^{N} is selected from the group consisting of hydrogen, optionally substituted C₁-₆-alkyl, optionally substituted aryl, optionally substituted heteroaryl, optionally substituted CH₂-aryl, as well as

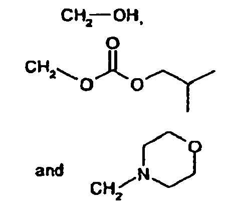

Illustrative embodiments of compounds of formula M demonstrating suitable embodiments of R^{N} are compounds of formula TT0046 (wherein R^{N} is H), TT0048, TT0051 and TT006.

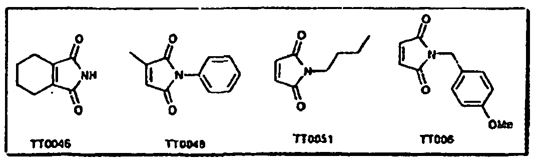

Further illustrative compounds, wherein R^{N} is an aliphatic amine linked to a ring system include M-i, M-li, M-lii, M-iv, M-v, M-vi and M-vii

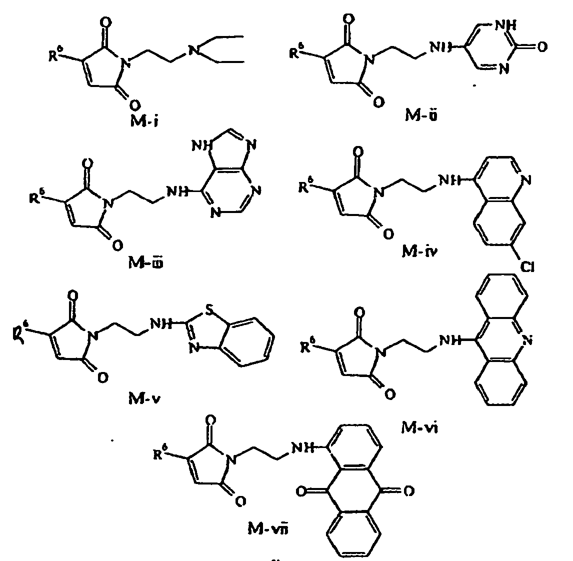

In a further suitable embodiments, R^{N} is an aliphatic amine wherein the amine nitrogen is part of ring system. Within such interesting embodiments comprises the embodiment comprising a maleimide dimer, linked through their respective imide nitrogens an aliphatic chain. The chain linking two ring systems may be of other chain lengths are further comprise functional groups and substituents, as is known to the person skilled in the art. Suitable embodiments comprise M-vill, M-ix, M-x, and M-xi.

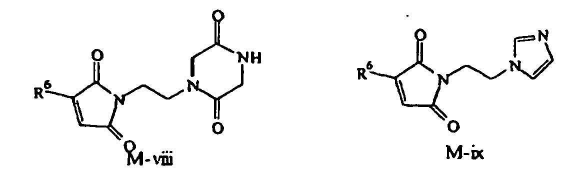

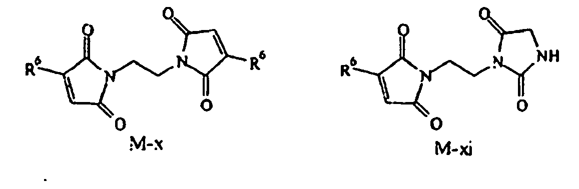

A particular interesting embodiment is wherein R^{N} comprises a heterocycle. The heterocyle may be substituted. In a suitable example, the heterocycle is substituted with a substituent which comprises a sulfoxide (SO₃H, SO₂), a hydroxy, a halogen, or any array of optional substituent as defined supra.

The rings of R^{N} may be substituted in a manner known to the person skilled in the art.
In a further preferred embodiment, at least one of R⁶ and R⁷ is hydrogen. In a combination of preferred embodiments, R¹ is preferably =O and at least one of R⁶ and R⁷ is hydrogen. In such an embodiment, the other of R⁶ and R⁷ is A-Y-Z, wherein A, Y and Z are as defined supra. In the embodiment wherein at least one of R⁶ and R⁷ is hydrogen and A is hydrogen, the compound of formula M is maleimide itself.

As can be seen from compound TT0046, in a suitable embodiment of compounds of formula M, R⁶ and R⁷ may together form a ring. Thus, R⁶ and R⁷ may together form a C₃₋₈₋carbocycle, heterocycyl, aryl or heteroaryl, each of which may optionally be substituted, preferably a C₃₋₈-carbocycle, such as cyclohexane.

In an interesting embodiment of compounds of formula M, O^{E} is =O, R¹ is =O, R^{N} is hydrogen, one of R⁶ and R⁷ is hydrogen, and the other of R⁶ and R⁷ is optionally substituted C₁₋₆-alkyl, such as selected from methyl (compound TT0043) or ethyl. Various tests were performed on compound TT0043 as illustrated in the Examples.

In the preferred embodiment wherein one of R⁶ and R⁷ is hydrogen and the other of R⁶ and R⁷ is A-Y-Z , A may be selected from the group consisting of hydrogen, -C(R²)(R²)-,--C(=O)-, -N(R^{N})-, -O-, -S-, -P-, -P(O)-; Y may be absent or selected from one of the group consisting of optionally substituted C₁₋₆-alkyl, optionally substituted C(=O)-C₃₋₆-alkyl, optionally substituted C₁₋₆-alkyl-C(=O), optionally substituted C₂₋₁₀-alkenyl, optionally substituted C₂₋₁₀-alkynyl, optionally substituted C₃₋₈-carbocycle and optionally substituted heterocycle; and Z is a monoradical selected from the group consisting of optionally substituted aryl, optionally substituted heteroaryl, optionally substituted C₃₋₈-carbocyle, optionally substituted heterocycle, H, OR^{Z}, N(R^{Z})(R³), S-R^{Z}, NO₂, -CN, and halogen.

In an interesting embodiment of compounds of formula M, one of R⁶ and R⁷ is hydrogen and the other of R⁶ and R⁷ is A-Y-Z wherein Z is selected from the group consisting of N(R⁸)(R⁹) and optionally substituted heteroaryl. In the suitable embodiment Z is an optionally substituted heterocycle, such as a nitrogen-, oxygen-, or sulfur-containing heterocycle. Preferably, the heterocycle is a nitrogen-containing heterocycle. In the embodiment wherein Z is a heterocycle, the heterocycle is preferably selected from the group consisting of maleimide, succinimide, hydantoin, thio-hydantoin, dioxypiperazine, dihydrouracil, imidazole, pyrazole, pyrrole, oxazole, furazan, barbituric acid, thiobarbituric acid, 3-alkoxyisoxazole, quinoline, aminoacridine, and cytosine, each of which may be optionally substituted. A particular interesting embodiment is wherein R^{N} comprises a heterocycle. The heterocyle may be substituted. In the suitable example wherein Z is a heterocycle, said heterocyle is optionally substituted with a substituent which comprises a sulfoxide (SO₃H, SO₂), a hydroxy, a halogen, an amine, or any array of optional substituent as defined supra.

In the embodiment wherein Z is a nitrogen-containing heterocycle, said heterocycle may be attached to Y by means of the nitrogen or by means of a carbon atom present in the heterocycle.

As stated, in an interesting embodiment of compounds of formula M, one of R⁶ and R⁷ is hydrogen and the other of R⁶ and R⁷ is A-Y-Z wherein Z is selected from the group consisting of N(R⁸)(R⁹), optionally substituted heteroaryl and optionally substituted heterocycyl. In the embodiment wherein Z is N(R⁸)(R⁹), R⁸ and R⁹ may independently be selected from the group consisting of hydrogen, optionally substituted C₁₋₆-alkyl, optionally substituted C₂₋₁₀-alkenyl, optionally substituted C₂₋₁₀-alkynyl, optionally substituted aryl, optionally substituted heteroaryl, optionally substituted heterocycle, optionally substituted C₃-C₇-cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³) and C(C=S)-N(R³)(R³). In a preferred embodiment, at least one of R⁸ and R⁹ is selected from the group consisting of optionally substituted heteroaryl and optionally substituted heterocycle, preferably an optionally substituted heterocycle. The present investigators have prepared compounds of formula M wherein 2 is N(R⁸)(R⁹) and one of R⁸ and R⁹ is an optionally substituted heterocycle is preferably selected from the group consisting of adenine, benzothiazole, maleimide, succinimide, hydantoin, thio-hydantoin, dioxypiperazine, dihydrouracil, imidazole, pyrazole, pyrrole, oxazole, furazan, barbituric acid, thiobarbituric acid, 3-alkoxyisoxazole, quinoline, aminoacridine, and cytosine, each of which may be optionally substituted. In the suitable example wherein one of R⁸ and R⁹ is an optionally substituted heterocycle, said heterocyle is optionally substituted with a substituent which comprises a sulfoxide (SO₃H, SO₂), a hydroxy, a halogen, an amine, or any array of optional substituent as defined supra.

As can be seen from Example 1, maleimide was surprisingly found to have topoisomerase II catalytic inhibitory activity. The present inventors have demonstrated that an array of the maleimide derivatives, have topoisomerase II catalytic inhibitory activity. In a most preferred embodiment, compounds of formula I are selected from compounds of formula M.

As stated, an important aspect of the present invention relates to the use of a compound of formula I for the manufacture of a medicament for treating diseases and disorders for which inhibition or pharmacologic modulation of the topoisomerase II enzyme produces a physiologically beneficial response in said disease or disorder. Example 1 demonstrates the activity of selected compounds of formula I as a topoisomerase II catalytic inhibitor. Correspondingly, the use of a compound of formula I for the preparation of a medicament for diseases or disorders for which inhibition or modulation of the topoisomerase II enzyme produces a physiologically beneficial response is an important aspect of the present invention. The use of a compound of formula M for the preparation of a medicament for diseases or disorders for which inhibition or modulation of the topoisomerase II enzyme produces a physiologically beneficial response is a particularly important aspect of the present invention.

As stated, the topoisomerase II enzymes belong to a family of nuclear enzymes involved in the processing of DNA during the cell cycle. In short, they are able to introduce transient cleavage of both strands of the DNA double helix, thereby allowing the passage of another intact DNA double strand through the cleavage. The duration of the transient DNA break is very short. Topoisomerase II catalytic inhibitors are known to be useful in the treatment of tumours. Thus, a further aspect of the present invention relates to the use of a compound of formula 1 for the manufacture of a medicament for treating a tumour in a mammal, such as a human, comprising administering to said mammal an effective amount of a compound of formula I. The use of a compound M for the manufacture of a medicament for treating a tumour in a mammal, such as a human, comprising administering to said mammal an effective amount of a compound of formula M is a particularly interesting aspect of the invention. Preferably, the anti-tumour effect results, at least in part, by inhibiting topoisomerase II enzyme. A related aspect of the present invention embodies the use of a compound of formula I for the preparation of a medicament for use in anti-cancer therapy. A preferred embodiment of this aspect relates to the use of a compound of formula M for the preparation of a medicament for use in anti-cancer therapy. Accordingly, the compounds of formula I, such as compounds of formula M, may be, in themselves, cytotoxic agents, anti-tumour agents or anti-cancer agents.

The compounds of the present invention may be particularly useful for the manufacture of a medicament for the treatment of tumours such as hypoxic solid tumours. Compounds of the present invention are anticipated to be particularly useful for the manufacture of a medicament the treatment of malignant melanoma, breast cancer, leukaemia and small cell lung cancer. Compounds of the present invention may also be particularly useful for the manufacture of a medicament for the treatment of tumours which are not especially enriched (poor) in their topoisomerase II concentration.

As stated, ICRF-187 is a topoisomerase II catalytic inhibitor. The present investigators have demonstrated that compounds of formula I are also topoisomerase II catalytic inhibitors. Topoisomerase II catalytic inhibitors such as ICRF-187 are useful for the prevention and treatment of accidental extravasation, such as due to topoisomerase poisons. It is anticipated that topoisomerase II catalytic inhibitors of formula I are useful for the prevention and treatment of accidental extravasation.

Thus, a further aspect of the present invention relates to the use of a compound of formula 1 for the manufacture of a medicament for preventing or treating tissue damage due to extravasation, such as due to topoisomerase II poisons, including anthracyclines, in a patient. A further aspect of the present invention relates to the use of a compound of formula I for the manufacture of an agent to prevent or treat extravasation.

In a suitable embodiment, the extravasation is the result of the administration of one or more topoisomerase II poisons. The topoisomerase poison responsible for the extravasation may be selected from the group comprising doxorubicin, daunorubicin, dactinomycin, epirubicin, bisantrene, pacitaxel, amsacrine, mitomycin C, vincristine, vinblastine, vindesine, liposomal anthracyclines, mitoxantrone, esorubicin, menogaril, acalcinomycin, cisplatin, fluorouracil, etoposide and bleomycin.

Moreover, the use of the catalytic inhibitors of topoisomerase II of the present invention may enhance the anti-cancer treatment of classical poisons resulting in a broader therapeutic index for the classical poisons by either redudng the side effects (toxicity) or by enhancing the effect of the poison.

A compound of formula I may be combined with an array of chemotherapeutic agents to provide an effective treatment of a variety of cancers.

However, a particularly interesting aspect of the present invention relates to the use of a compound of formula I, preferably M, for the manufacture of a medicament which, when combined with a topoisomerase II poison, is effective in the treatment of cancer and to the use of a combination of topoisomerase II poison and a compound of formula I, such as a compound of formula D or M, preferably M, for the manufacture of a medicament for anti-cancer therapy in a of mammal, such as a human.

The combination treatment may involve the combining of one or more compounds of formula I, preferably M, with a chemotherapeutic agent be selected from the group comprising pacitaxel, doxorubicin, daunorubicin, dactinomycin, epirubicin, bisantrene, pacitaxel, amsacrine, mitomycin C, vincristine, vinblastine, vindesine, liposomal anthracyclines, mitoxantrone, esorubicin, menogaril, acalcinomycin, cisplatin, fluorouracil, etoposide and bleomycin, mithramycin, melphalan, carmustine, darcabazine, cytarbine, methotrexate, teniposide, L-asparaginase, alfa-interferon, interleukin 2 and other chemotherpeutic agents known the person skilled in the art, preferably pacltaxel, doxorubicin, daunorubicin, dactinomycin, epirubicin, bisantrene, pacitaxel, amsacrine, mitomycin C, vincristine, vinblastine, vindesine, liposomal anthracyclines, mitoxantrone, esorubicin, menogaril, acalcinomycin, cisplatin, fluorouracil, etoposide and bleomycin.

Cancers considered to be especially suitable for the combination treatment may be selected from the group comprising malignant melanoma, breast cancer, leukaemia and small cell lung cancer.

As is known to the person skilled in the art, salts of compounds of formula I, M, such as quaternary ammonium salts, are embodied in the present invention. Moreover, enantiomeric, diastereomeric and racemic forms of compounds of the invention are also anticipated.

A further aspect of the present invention relates to a pharmaceutical composition comprising at least one compound selected from the group consisting of formula I, M, with at least one pharmaceutically acceptable excipient or carrier further comprising one or more chemotherapeutic agents selected from the group comprising pacitaxel, doxorubicin, daunorubicin, dactinomycin, epirubicin, bisantrene, pacitaxel, amsacrine, mitomycin C, vincristine, vinbiastine, vindesine, liposomal anthracyclines, mitoxantrone, esorubicin, menogaril, acalcinomycin, cisplatin, fluorouracil, etoposide and bleomycin, mithramycin, melphalan, carmustine, darcabazine, cytarbine, methotrexate, teniposide, L-asparaginase, alfa-interferon, interleukin 2 and other chemotherpeutic agents known the person skilled in the art, preferably pacitaxel, doxorubicin, daunorubicin, dactinomycin, epirubicin, bisantrene, amsacrine, mitomycin C, vincristine, vinblastine, vindesine, liposomal anthracyclines, mitoxantrone, esorubicin, menogaril, acalcinomycin, cisplatin, fluorouracil, etoposide and bleomycin.

In a particularly preferred embodiment, pharmaceutical compositions comprise one or more compounds of formula M and further comprise one or more topoisomerase II poisons. Pharmaceutical compositions may be suitably formulated for oral, mucosal, intravenous, transdermal, parenteral or intracranial administration.

### BRIEF DESCRIPTION OF THE FIGURES

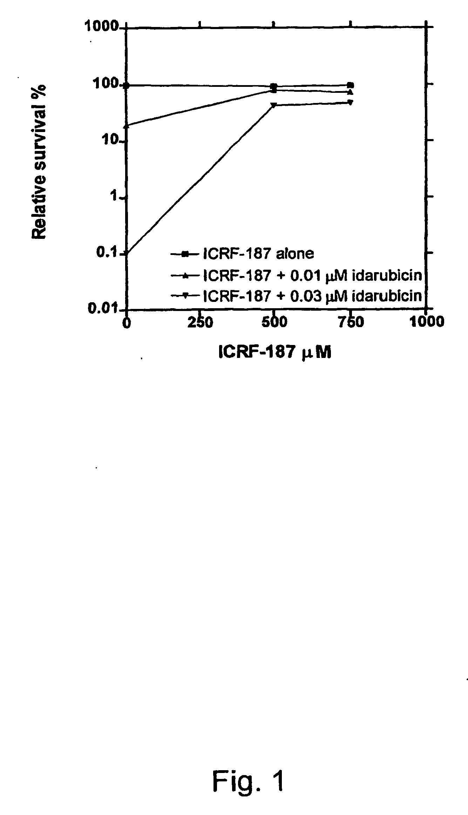

The effect of the compounds of the present invention on the cytotoxicity of some of the classical poisons is illustrated in Figures 1 to 3 showing the results from the clonogenic assays.

In Figure 1 the effect of ICRF-187 on idarubicin induced cytotoxicity is shown. As seen from the figure, ICRF-187 shows no toxicity to cells when given alone. Idarubicin was used in two different concentrations (0.01µM and 0.03µM). Clearly ICRF-187 was able to inhibit the toxicity induced by idarubicin (0.01mM) up to about 80% survival. Likewise, ICRF-187 has been shown to inhibit the cell toxicity of several other poisons as well.

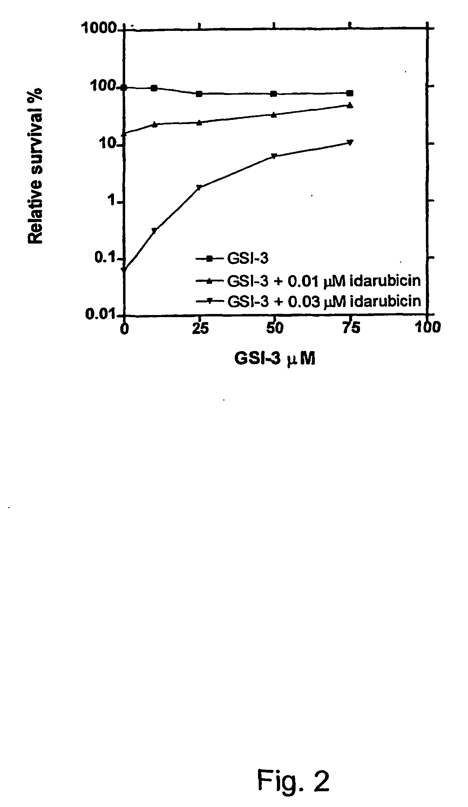

Figure 2 shows the inhibitory effect of the compound I-112 (identical with GS-I-3) on idarubicin cytotoxicity (same concentrations as mentioned above). I-112 shows no cytotoxicity on its own, but is clearly able to protect against poison-induced cell damage. As seen, the concentrations of compound I-112 used are lower than for ICRF-187 (I-112 is thus more potent than ICRF-187 in antagonizing the effect of the topoisomerase II poison idarubicin), while maximum protection is less than seen for the lead compound ICRF-187.

Figure 2 thus demonstrates the ability of compounds of formula I to attenuate the toxic effects of classical poisons such as the prevention of poison-induced cell damage, i.e. extravasation.

Figure 3 shows the effective inhibition of poison induced toxicity by the compound of formula M, maleimide. The compound was tested against etoposide (VP-16, in 20µM concentration) and daunorubicin (in 0,3 µM concentration). In both cases, the inhibition of cell toxicity was strong, but maleimide is also toxic to cells in itself.

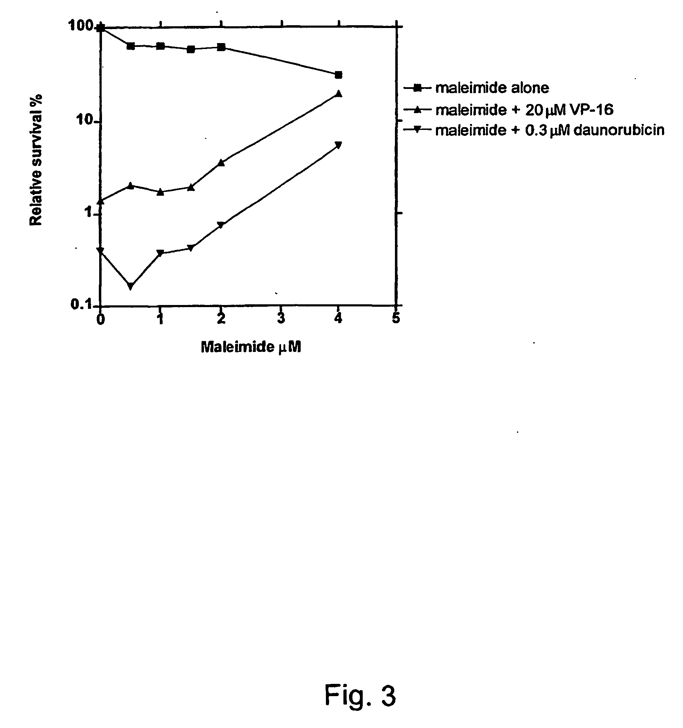

Figure 3 thus further demonstrates the ability of compounds of formula M to attenuate the toxic effects of classical poisons such as the prevention of poison-induced cell damage, i.e. extravasation.

Figure 3 also demonstrates the ability of compounds of formula M to act as anti-tumour (anti-cancer) agents in themselves.

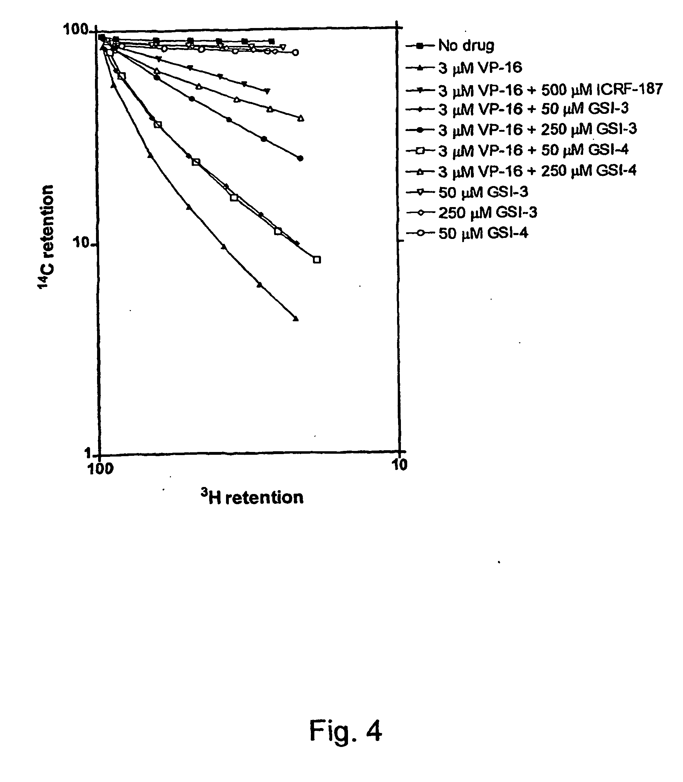

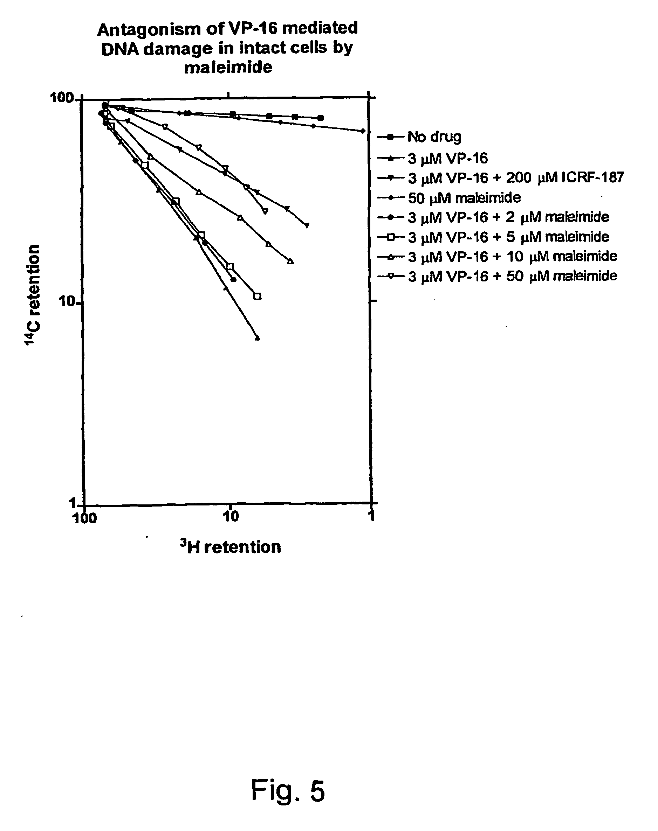

The effect of the compounds of the present invention on DNA damage conferred by classical poisons is illustrated on Figure 4 and 5 using selected compounds.

As an example, the alkaline elution assay on Figure 4 shows the reversal of etoposide (VP-16) induced SSBs by ICRF-187 and by the compound I-112 (identical with GS-I-3). The promoting effect of etoposide (3µM) on DNA damage is shown on curve 2. The effect of ICRF-187 (500µM) and of I-112 (250µM) when tested against etoposide is seen in the curves 3 and 5, respectively.

Clearly, these catalytic inhibitors of topoisomerase II are able to antagonise the SSBs induced by etoposide.

Figure 5 illustrates the ability of maleimide to antagonise etoposide-induced DNA damage in intact cells. Curve 1 is a no drug control, the etoposide effect alone is seen from curve 2. The ability of compound 1-1 (50 µM) to antagonise this etoposide (VP-16) effect is seen on curve 8. This antagonism is similar to the antagonism shown for the lead compound ICRF-187.

Figure 4 thus further demonstrates the ability of compounds of formula I, particularly those of formula M, to attenuate the toxic effects of classical poisons such as the prevention of poison-induced cell damage, i.e. extravasation.

Figure 5 thus demonstrates the ability of compounds of formula M (maleimide derivatives) to attenuate the toxic effects of classical poisons such as the prevention of poison-induced cell damage, i.e. extravasation.

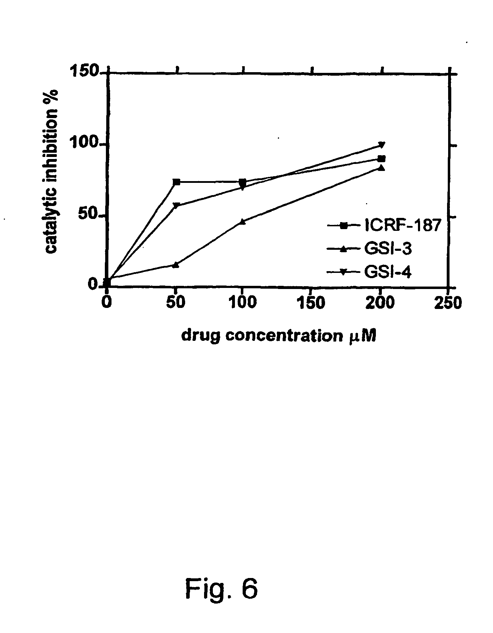

Figure 6 illustrates the effect of one of the compounds of the present invention (I-112, identical with GS-I-3) on topoisomerase N catalytic activity using the decatenation assay. Like the control (ICRF-187) compound I-112 is able to inhibit the topoisomerase II catalytic activity effectively at pharmacologically relevant concentrations.

Figure 6 thus demonstrates the ability of compounds of formula I to act as anti-tumour (anti-cancer) agents in themselves.

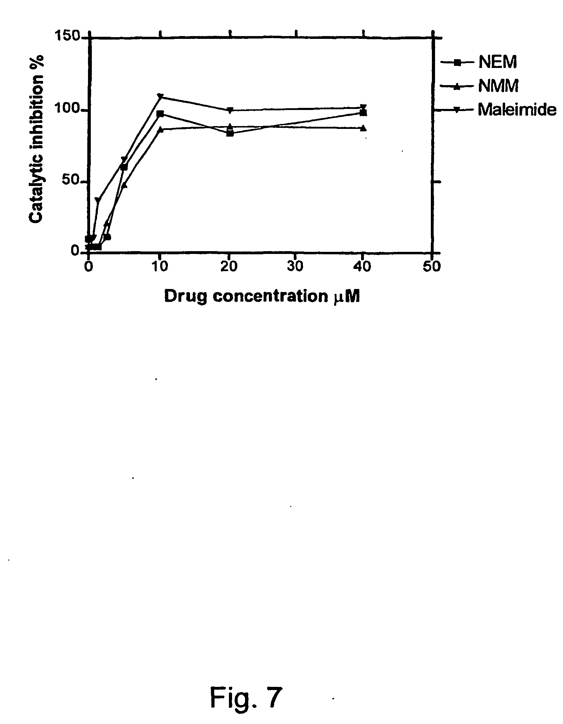

Figure 7 shows the result from a decatenation assay performed with maleimide, NMM and NEM. Clearly, these compounds are highly active on the enzyme having an IC₅₀ of about 2µM. for Maleimide and about 4 µM for NMM and NEM.

Figure 7 thus demonstrates the ability of compounds of formula M to act as anti-tumour (anti-cancer) agents in themselves.

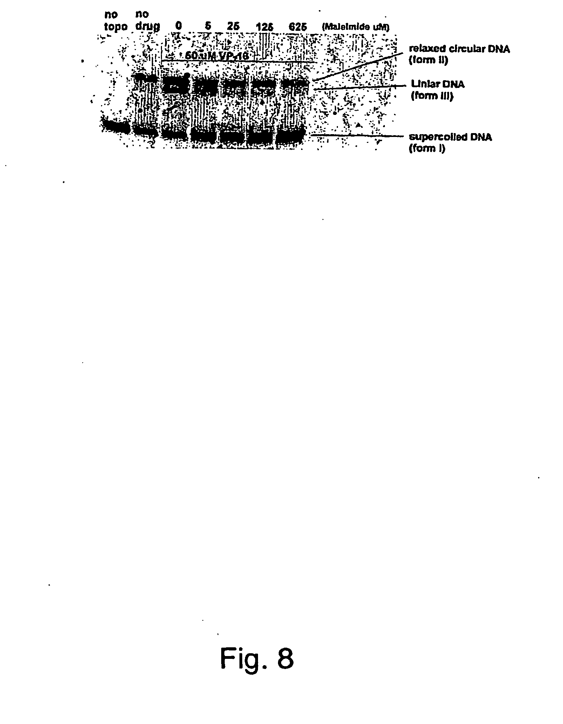

Figure 8 shown the result of a plasmid cleavage assay. Maleimide is found to antagonize etoposide induced formation of liniar DNA (form III) at concentrations between 5.0 and 625 µM.

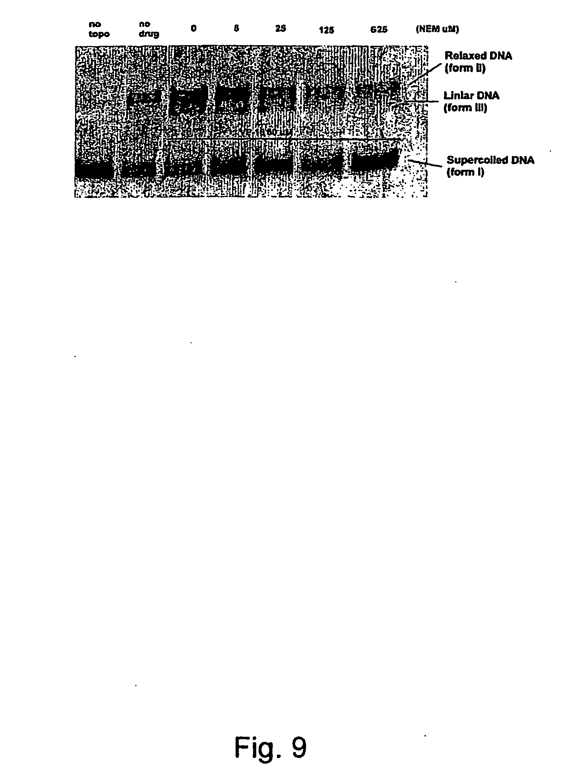

Figure 9 shows a similar effect of NEM on etoposide induced formation of linear DNA.

Figure 8 and 9 thus demonstrates the ability of compounds of formula M to act as pharmacological regulators of classical topoisomerase II poisons by antagonizing the effect of these.

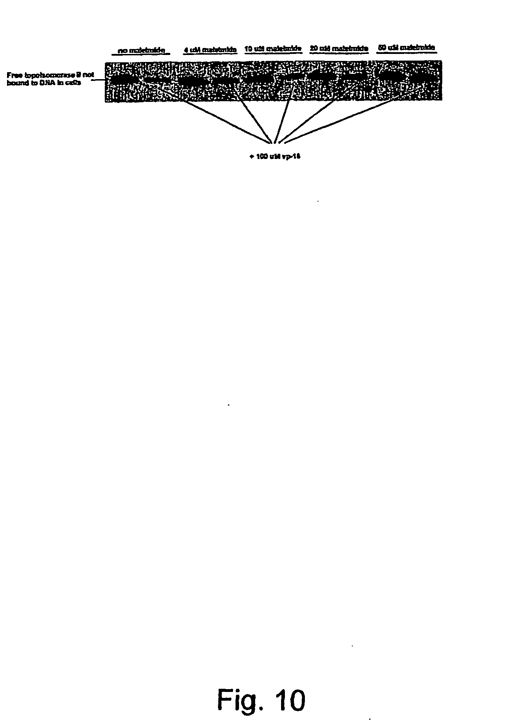

Figure 10 demonstrates the ability of a compound of formula M, maleimide, to antagonize etoposide induced band depletion of cellular topoisomerase II enzyme. It is seen that the extractable amount of topoisomerase II is clearly reduced when cells are treated with 100 µM etoposide. When maleimide is coapplied this effect of etoposide is gradually lost. At 50 µM maleimide the band depleting effect of etoposide is completely lost.

Figure 10 thus demonstrates the ability of compounds of formula I to attenuate the effects of classical topoisomerase II poisons allowing for pharmacological regulation of these.

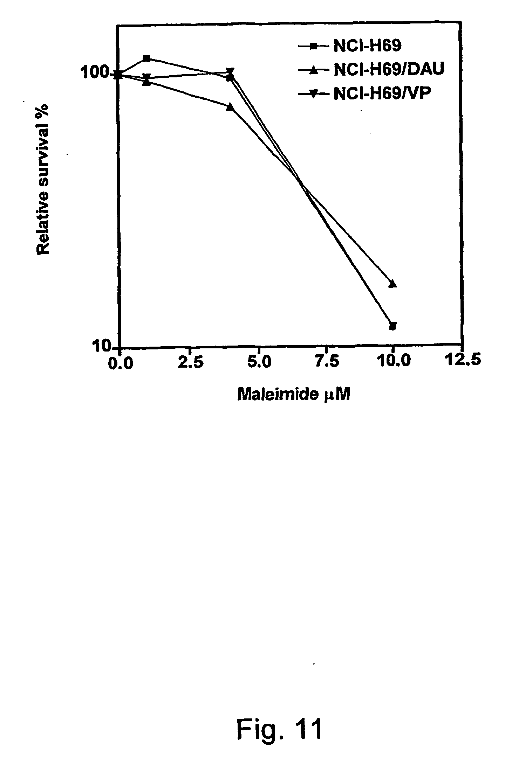

Figure 11 demonstrates the cytotoxicity of a compound of formula M, maleimide, on the human small cell lung cancer cell line NCl-H69 and on its multi drug resistant derivatives NCl-H69/DAU and NCl-H69/VP. No cross resistance is observed as the three cell lines are equally sensitive to malemide.

Figure 11 thus demonstrates the ability of compounds of formula M to act as anti-tumour (anti-cancer) agents in themselves circumventing the MDR phenotype of cancer cells.

### EXAMPLES

### Example 1: Pharmacological Activity of Selected Compounds

### Pharmacological Assays

The *in vitro* and *in vivo* pharmacological assays used to characterise the compounds to be claimed as catalytic inhibitors of the topoisomerase II enzyme are as follows: Clonogenic assay, decatenation assay, alkaline elution, band depletion and plasmid cleavage assay. These assays cover a range of information and shall, as proof of concept of this class of compounds for the use as catalytic inhibitors of topoisomerase II, very briefly be described:

### Clonogenic assay

The information derived from the clonogenic assay is cytotoxicity. If a given compound is able to antagonize the cytotoxic effect caused by the interaction of cellular topoisomerase II and classical topoisomerase II poisons, the compound is classified as a catalytic topoisomerase II inhibitor (CI) For medical use, the catalytic inhibitors should only be cytotoxic in relatively high concentrations by themselves.

A 3-week clonogenic assay is used. Briefly, single cell suspensions (2 × 10⁴ cells/mL) in RPMI 1640 supplemented with 10% fetal calf serum are exposed to the drugs for 1 h; washed twice in PBS at 37°C, and plated in triplicate in soft agar on top of a feeder layer containing sheep red blood cells. The number of cells is adjusted to obtain 2000 to 3000 colonies in the control dishes. Finally, the colonies are counted.

### Decatenation assay

The decatenation assay is a direct measurement of a given compounds inhibitory action on purified topoisomerase II enzyme. The general role of the topoisomerase II enzyme in living cells is to cause decatenation of catenated DNA during cell division. If a compound is able to inhibit this decatenation, the compound is an inhibitor of the topoisomerase II enzyme. While this assay is capable of determining whether a given compound is active against topoisomerase II, information concerning the exact mode of action is not provided.

Topoisomerase II catalytic activity is measured by kDNA decatenation. ³H labelled kDNA is isolated from Crithidia fasciculata (ATCC, Manassas, VA) as described in (24). Briefly, relevant concentrations of the test compound in buffer (50mM Tris-Cl, pH8, 120mM KCl, 10mM MgCl₂, 1.0mM ATP, 30mg BSA/ml) and purified topoisomerase II α is incubated with 0.2 mg kDNA for 15 min at 37°C in a final volume of 20 µl. After addition of stop buffer/loading dye mix (5% Sarkosyl, 0.0025% bromophenol blue, 25% glycerol), samples are loaded on 1% agarose/0.5% ethidium bromide gels and run in TBE buffer containing 0.5 mg/ml ethidium bromide at 100V for approximately 50 min and photographed under UV light. In addition, loading wells are cut out and scintillation counted to obtain numerical values. These values are then plotted against drug concentration to obtain dose-response curves enabling the determination of IC₅₀ values for individual compounds.

### Alkaline elution

In alkaline elution, which is a whole cell assay (in vitro assay), the compounds are tested in cells for their ability to interfere with the formation of single strand DNA breaks (SSBs) induced by classical poisons e.g. etoposide and daunorubicin. This assay thus provides information concerning a given compounds ability to cause (or antagonize in the case of topoisomerase II catalytic inhibitors in the combination with classical topoisomerase II poisons) DNA damage in intact cells.

The assays for SSBs and for DPCs (DNA-protein cross-links) are performed according to Kohn et. al (23).

For measurement of single-strand breaks (SSBs), L1210 cells used as internal standard are exposed to 100 µM H₂O₂ for 60 min on ice, corresponding to an irradiation dose of 300 rad as described by Szmigiero and Studzian (25). OC-NYH cells are incubated in medium supplemented with the indicated drug at 37°C for the specified periods, washed in 10 mL ice-cold PBS and then lysed on the filter (Nucleopore, 2.0 µM pore size) with 5 mL of SDS-EDTA lysis solution (2% SDS, 0.1 glycine and 0.025 M Na₂EDTA) at pH 10, followed by addition of 1.5 mL SDS-EDTA lysis solution supplemented with 0.5 mg/mL proteinase K. Mixing of standard and experimental cells is done immediately prior to lysis. DNA was eluted with tetrapropyl-ammoniumhydroxide-EDTA, pH 12.1 containing 0.1% SDS at a rate of 0.125 mL/min. Fractions are collected at 20-min intervals for 2 h. Filters are treated with 400 µL 1 N HCl for 1 h at 60°C, cooled and 0.4 M NaOH Is added prior to scintillation counting.

Compounds capable of antagonizing the DNA damage induced by dassical topoisomerase II poisons such as etoposide are classified as topoisomerase II catalytic inhibitors.

### Band depletion

The band depletion assay is also related to the mode of action of the compounds at the enzyme level in intact cells. Incubation of the cell, with e.g. etoposide, reduces the amount of extractable topoisomerase II α enzyme in the cells (27). Thus by inhibiting religation, etoposide traps the enzyme on its DNA substrate, leaving less freely available enzyme for salt extraction. Experiments with the catalytic inhibitor ICRF-187 (preincubated 1 h) has shown (8) that the compound is also able to induce a marked decrease (ATP-dependent) in the extractable amount of both topoisomerase II α and β isozymes. A correlation between these results and the mode of action of the bisdioxopiperazines acting by locking the homodimeric topoisomerase II in the form of a closed bracelet surrounding the DNA at the post-ligation step has been demonstrated.

OC-NYH cells are incubated for 1 h at 37C In RPMI 1640 with 10% of fetal calf serum with the desired compounds for test. Thereafter, whole cell lysates were obtained at 4C. Exponentially growing cells were harvested and washed in PBS. Cells are resuspended in 0,2 mL ELB-buffer (NaCl 0,25 M, NP40 0,1%, HEPES 50mM, EDTA 5mM) containing aprotinin 1µg/ml, leupeptin 1 µg/ml, DTT 1µM and PMSF 0,3 mM and lysed for 30 min-Pellets are spun down at 20000 × *g* for 20 min. The protein concentration of the supernatants is measured, and the supernatants are diluted with an equal volume of glycerol.

After heating the nuclear extracts for 5 min at 95C, the sample is immediately loaded on a 7% SDS-PAGE gel containing 5% glycerol. Thereafter, all steps are performed at RT. The separated proteins are transferred to Trans-Blot**®** Nitrocellulose, in a Mini Trans-blot**®** Electrophoretic Transfer Cell with 25mM Tris, 192mM glycine and 205v/v methanol, pH 8,3 at 210 mA for 1 h. Membranes are blocked in 10% milk in TBS-T for 1 h and probed with either topoisomerase II α (1:1000) 1 h. Horseradish-peroxidase-conjugated donkey-antirabbit antibodies are used as secondary antibodies. The blots are developed using ECL™reagent 1 min and thereafter exposed on films for 5 min. Quantification of can be made by densitometric scanning.

While this assay in not capable of demonstrating directly whether a given compound is a topoisomerase II catalytic Inhibitor (ICRF-187 and poisons both induce band-depletion) compounds capable of antagonizing the band depletion effect of classical topoisomerase II inhibitors such as etoposide are classified as topoisomerase II catalytic inhibitors.

### Plasmid Cleavage assay

The plasmid cleavage assay is capable of assessing wether a given compound stimulates topoisomerase II mediated DNA deavage in the test tube. A modification of the protocol described in (Burden *et al.,* 2001) is used. 350 ng purified human topoisomerase II o, 400 ng pUC18 DNA, and increasing concentrations of drugs were incubated for 6 min at 37 °C in 20 µl topoisomerase II cleavage buffer (10 mM TRIS-HCL pH 7.9, 50 mM NaCl, 50 mM KCl, 5 mM CaCl₂, 1 mM EDTA, 15 µg/ml BSA and 1 mM Na₂ATP, all from Sigma Chemical Co., St. Louis, MO, USA). Next, the cleavable complex was trapped by adding 2 µl 10 % SDS. After vigorous vortexing 1.5 µl 0.25 M EDTA and 2 µl proteinase K (0.8 µg/ml) in proteinase buffer (50 mM Tris-HCL pH = 7.9, 1 mM CaCl₂) was added and the samples vortexed. After a 30 min incubation at 45 °C, 5 µl loading buffer (5% Sarkosyl, 0.0025% bromophenol blue, 25% glycerol) was added and the samples were exposed to 70 °C for 5 min. Next, samples were run through a 0,8 % agarose gel in 1 X TAE buffer for three hours at 5 V/cm to separate different topological forms of plasmid DNA. Finally the gels were strained in distilled water containing 10 µg/ml ethidlum bromide for 15 min followed by de-strained in distilled water for one hour before they were photographed in UV light. While some classes of topoisomerase II catalytic inhibitors like the bisdioxopiperazine compounds are not capable of antagonizing the effect of classical topoisomerase II poisons in this assay, any compound capable of doing so such as Maleimide and NEM is classified as a topoisomerase II catalytic inhibitor. Further, compounds not capable of stimulating topoisomerase mediated DNA cleavage while being active in the decatenation assay are also classified as topoisomerase II catalytic inhibitors.

### Pharmacological Activity of Selected Compounds

The compounds to be claimed as novel catalytic inhibitors of topoisomerase II have been tested in the pharmacological assays mentioned above. Pharmacological data of selected compounds to be claimed are given below together with a brief explanation of the results. This is to verify the use as catalytic inhibitors of this class of compounds. Data are shown for the bisdioxopiperazine ICRF-187 (for comparison) as well as for the compounds I-21, I-112 and maleimide.

| | **ICRF-187** | **I-21** | **I-112** |
|---|---|---|---|
| **Clonogenic Assay** | LD₅₀ = 75µM. Not toxic to cells. Effective inhibitor of the toxicity of the classical poisons (VP16, DAU, IDA). Ex: at 500µM (IDA(0.01)+ up to 80% survival) and (IDA(0.03)++ up to 40% survival)* | LD₅₀ = 750µM. Not toxic to cells at pH7.4. Tox at lower pH. Only very weak protection of poisons. Ex: at 500µM (VP-16(20µM)+ up to 1% survival). | LD₁₀ = 75µM. Not toxic to cells. Inhibits the toxicity of poisons (DAU, IDA) at lower conc. than ICRF-187 (more potent), but is weaker in the protection. VPneg. Ex: at 75 µM (IDA(0.03 µM)++ up to 10% survival and DAU(0.3 µM)+ up to 20% survival. |
| **Decatenation Assay** | Inhibits the topoisomerase II decatenation effectively. IC₅₀ about 50 µM. | Inhibits the topoisomerase II decatenation from above 500µM. IC₅₀ about 800 µM. | Inhibits the topoisomerase II decatenation effectively. IC₅₀ about 100 µM. |
| **Alkaline Elution** | Effective inhibition of VP-16 induced SSBs at 200µM. | No effect on the VP-16 induced SSBs found from 50-500µM. | Effective inhibition of VP-16 induced SSBs at 250 µM. Very similar to ICRF-187 profile. |
| **Band depletion** | Reversible ATP dependent decrease in the extractable levels of both topoisomerase II aand b. Preincubated 50µM. | n.d. | n.d. |
| Plasmid Cleavage assay | No stimulation of topoisomerase II mediated DNA cleavage. No antagonism of etoposide induced DNA cleavage. | n.d | n.d. |

| | | | |
|---|---|---|---|
| *) At a concentration of poison, here ldarubicin, of 0.01µM, ICRF-187 (500µM) is capable of protecting the cells so as to obtain 80 % of survival (maximum protection at the given dose). The + indicates, that the protection. i.e. log(number of surviving cells) is about 1. | | | |

| | maleimide | NMM | NEM | TT0043 |
|---|---|---|---|---|
| Clonogenic assay | LD₅₀ 4µM. Very toxic to cells. Effective inhibitor of the classical poisons; Like ICRF-187. Ex: at 0.5µM (VP-16 (20µM)+ up to 30% survival) and DAU (0.5µM)++ up to 10% survival. | IC50 4 µM. Antagonism of etoposide and daunorubicine mediated cytotoxicity is equal to what is observed with Maleimide. | IC₅₀ 4 µM. No antagonism of etoposide and daunorubicin mediated cytotoxicity is observed | LD₅₀ = 40 µM. Much less toxic than melaimide, NMM and NEM. Protects against the cytotoxicity conferred by 10 µM etoposide and 0,3 µM daunorubicin. |
| Decatenation | Very potent, inhibiting topoisomerase II catalytic activity with IC50 of 2 µM. | Very potent, inhibiting topoisomerase II catalytic activity with IC50 of 5 µM. | Very potent, inhibiting topoisomerase II catalytic activity with IC50 of 5 µM. | n.d. |
| Alkaline elution | Effective inhibition of VP-16 induced SSBs at 50µM (better than ICRF-187 at 200µM) Only limited DNA damage at concentrations up to 250 µM | n.d. | Only limited DNA damage at concentrations up to 250 µM. Antagonism og topoisomerase II poisons not determined | n.d. |
| Band depletion | Decrease in the extractable levels of topoisomerase II Similar profile as for ICRF-187. (50µM). Antagonism of etoposide (100 µM) induced band depletion between 4 and 50 µM | n.d | n.d | n.d. |
| Plasmid cleavage assay | No evidence of topoisomerase II mediated DNA cleavage up to at concentrations up to 10 mM. Strong antagonism of etoposide (50 µM) induced DNA cleavage between 5 and 625 µM | n.d. | No evidence of topoisomerase II induced DNA cleavage at concentrations up to 10 mM. Strong antagonism of etoposide (50 µm) induced DNA deavage between 5 and 625 µM | n.d. |

### Example 2: Synthesis of Sample Compounds

Compounds of the invention may be prepared by the following synthesis:

### General

Maleimides may be prepared in a one-step reaction from readily available anhydrides when treated with a HMDS/methanol reagent in a DMF solution at room temperature.

### TT0043

Compound TT0043 has been synthesised using the general procedure using citraconic anhydride as starting material. 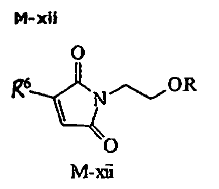 Compounds of the Type M-xii (R⁶ = Me as an example) is made using 3-methyl maleic anhydride and ethanol amine as starting materials. Ring closure using acetic anhydride affords the primary alcohol. The alcohol can be converted to various ethers or esters using conventional methods.

### M-xiii

The synthesis of the compounds of formula M-xiii was done in a series of reactions with 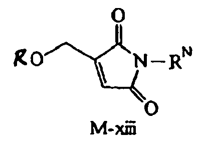
maleimide as starting material. Initial bromination of the double bond gives 3-bromo-maleimide. Subsequent protection of the carbonyl groups followed by n-BuLi and formalin and deprotection results in 3-hydroxymethyl-maleimide. This compound gives rise to a number of reactions. This alcohol can be converted to various ethers or esters using conventional synthetic methods.

Compound (3) can be made, as described above, from a substituted maleic anhydride and ethanol amine. Ring closure to the maleimide structure affords a compound (primary alcohol) which can, upon Mitsunobu reactions using appropriate substituted phenols, give the wanted structure.

### Compounds of the type M-i to M-vii

Compounds of the type M-i to M-vii may be prepared as described for compounds of the type M-xiii using a Mitsunobu coupling reaction to the amino-substituted ring moiety (such as aniline).

Compound M-i was prepared in the following manner:
The primary alcohol (1-(2-Hydroxy-ethyl)-3-methyl-pyrrole-2,5-dione) the synthesis of which is described above, is converted to an alkyl bromide using conventional methods. This bromide can be coupled to various amines affording the structures mentioned above. Reaction of this bromide with, as an example, diethyl amine, gives the compound M-i.

Compound M-ii to M-vi were prepared as described for M-i using instead cytosine, adenine, hydantoine, thiohydantoine imidazole, benzothiazole, aminoacridin or quinoline can give the desired compounds.

### N,N'-Di-(pyrrolidine-2',5'-dione-3'-yl)-ethylene-1,2-diamine (I-21)

6.11 g Maleimide (63 mmol) was dissolved in absolute ethanol (75 ml). 2 ml Ethylendiamine (30 mmol) was quickly added at RT. After approximately 5 min., a white precipitate appeared. After 18 hours, the product was collected by filtration, yielding 1.13 g (14.8%) of the title compound.

### N,N'-Di-(pyrrolidine-2',5',-dione-3'-yl)-propylene-1,3-diamine (I-14)

0.49 g Maleimide (5 mmol) was dissolved in freshly distilled THF (10 ml). 167µl Propylene-1,3-diamine (2 mmol) was added drop wise to the solution. After a short while, the mixture became turbid. After 2 hours, the mixture was evaporated to give an oil. The product was purified by flash chromatography (20% methanol/DCM) to yield 0.35 g (1.3 mmol, 64%) of *N,N*'-di-(pyrrolidine-2,5-dione-3-yl)-propylene-1,3-diamine as an oil. ¹H NMR (300MHz, DMSO-d₆) δ 11.06 (s, 2H, 2ximid), 3.63 (dd, 2H, 2xCOCH(CH₂)N), 2.81 (dd, 2H, 2xCOCHHCH), 2.70 (m, 2H, 2xNCHHCH₂), 2.50 (m, 2H, 2xNCHHCH₂), 2.33 (dd, 2H, 2xCOCHHCH), 1.52 (m, 2H, CH₂CH₂CH₂). ¹³C NMR (75.5MHz, DMSO-d₆) δ 180.0, 177.1 (C₂·, C₅·), 57.1 (C₃·), 44.9 (C₁, C₃), 37.0 (C₄·), 29.9 (C₂). FAB+ 269.13.

### 3-N-Butylamino-pyrrolidine-2,5-dione (I-10)

0.37 g n-Butyl amine (5 mmol) was dissolved in ethyl acetate (5 ml). The solution was cooled to 0°C and 0.48 g maleimide (5 mmol) dissolved in ethyl acetate (15 ml) was added drop wise. After 18 hours, the solvent was evaporated and the product purified by flash chromatography (5% methanol/DCM) to yield 0.82 g (4.82 mmol, 96%) of 3-*N-*butylamino-pyrrolidine-2,5-dione as an oil. ¹H NMR (300MHz, CDCl₃) δ 3.82 (dd, 1H, COCH(CH₂)N), 2.95 (dd, 1H, COCHHCH), 2.55-2.73 (m, 3H, NCH₂CH₂, COCHHCH), 1.49 (m, 2H, CH₂CH₂CH₂), 1.38 (m, 2H, CH₂CH₂CH₃), 0.92 (t, 3H, CH₃).

### 3-N-Isopropylamino-pyrrolidine-2,5-dione (I-11)

0.30 g Isopropyl amine (5 mmol) was dissolved in ethyl acetate (5 ml). The solution was cooled to 0°C and 0.48 g maleimide (5 mmol) dissolved in ethyl acetate (15 ml) was added drop wise. After 48 hours, the solvent was evaporated and the product purified by flash chromatography (5% methanol/DCM) to yield 0.73 g (4.67 mmol, 93%) of 3-*N-*isopropylamino-pyrrolidine-2,5-dione as an oil. ¹H NMR (300MHz, CDCl₃) δ 3.87 (dd, 1H, COCH(CH₂)N), 2.92-2.99 (m, 2H, COCHHCH, NCH(CH₃)₂), 2.95 (dd, 1H, COCHHCH), 1.11 (t, 3H, CH₃).

### 4,5,6,7-Tetrahydro-isoindole-1,3-dione (TT0046).

A solution of 3,4,5,6-tetrahydrophtalic anhydride (330 mg, 2.17 mmol) in DMF (5 ml) was treated with a mixture of HMDS (4.6 ml, 21.7 mmol) and methanol (0.5 ml, 10.8 mmol). After 16h at RT the mixture was poured into water and extracted with ethyl acetate. The combined organic layers were washed with water (3 × 12 ml), dried (MgSO₄), and filtered. Purification by column chromatography using a gradient of 1:1 to 3:1 EtOAc:hexane to give 158.3 mg (48%) of 2: ¹H NMR (300 MHz, DMSO-d₆) **δ** 10.47 (broad s, 1H, NH), 2.88 (m, 4H), 2.72 (m, 4H).

### 3-Methyl-1-phenyl-pyrrole-2,5-dione (TT0048).

A solution of citraconic anhydride (3.74 g, 33.4 mmol) and diethyl ether (4 ml) was placed in a 250 ml three-necked flask provided with a reflux condenser. A solution of aniline (3.11 g, 33.4 mmol) and ethyl ether (3 ml) was added drop wise. The resulting thick suspension was stirred at RT for 1h and was then cooled In an ice bath. Filtration and drying of the product *in vacuo* gave 6.31 9 (92%) of 3-methyl-3-phenylcarbamoyl-acrylic acid, which was used without further purification. A solution of crude 3-methyl-3-phenylcarbamoyl-acrylic acid (3.00 g, 14.6 mmol), acetic acid (30 ml) and anhydrous sodium acetate was dissolved by swirling and heating in an oil bath for 30 minutes. The reaction mixture was cooled to RT and was poured into 30 ml of ice water. The product precipitated. Filtration, washing of crystals using cold water (3 × 25 ml), cold n-heptane, drying *in vacuo* and purification by column chromatography (ethyl acetate) gave **TT0048** (601 mg (21%) as white needles. ¹H NMR (250 MHz, DMSO-d₆) δ 7.40 (m, 2H), 7.21 (m, 2H), 7.00 (m, 1H), 6.80 (m, 1H), 2.06 (broad s, 3H). LCMS: 188 (M+1).

### 1-Butyl-pyrrole-2,5-dione (TT0051)

3-Butylcarbamoyl-acrylic acid (864mg; 5,047mmol) was dissolved in acetic anhydride (9ml) containing sodium acetate (800mg). The solution was heated to reflux for 30 min. cooled to RT and ice-cold water (30ml) was added. The mixture was stirred for 10 min, extracted (ethyl acetate × 3), dried (MgSO₄), evaporated *in vacuo* to afford the crude product (810mg). The residue was purified by column chromatography (EtOAc), and gave the title compound **TT0051** (460 mg, 60%) as an oil. ¹H NMR (300MHz, CDCl₃) δ 6.70 (s, 2H), 3.52 (t, 2H), 1.6 (m, 2H), 1.3 (m, 2H), 0.95 (t, 3H). LC-MS: 154 (M+1).

### 1-(4-Methoxy-benzyl)-pyrrole-2,5-dione (TT0006).

Maleic anhydride (3.02 g, 30.6 mmol) was dissolved in THF (5 ml). 4-Methoxyphenylamine (4 ml, 30.6 mmol) was added, and the mixture was heated to reflux for 2h. The resulting suspension was cooled to RT, filtered, washed with cold THF (2 × 10 ml), and dried *in vacuo* to give **TT0006** (5.68 g, 85%) ¹H NMR (300 MHz, DMSO-d₆) δ 9.40 (broad s, 1H, NH), 7.24 (d, 2H), 6.90 (d, 2H), 6.45 (d, 1H, *J* = 12 Hz), 6.26 (d, 1H, *J* = 12 Hz), 4.32 (d, 2H, *J* = 5 Hz), 3.73 (s, 3H).

### References

(1) Bork E, Ersbell J, Dombernowsky P, Bergman B, Hansen M, Hansen HH (1991) Teniposide and etoposide in previously untreated small-cell lung cancer: A randomised study. J Clin Oncol 9: 1627.
(2) Froelich-Ammon SJ, Osheroff N (1995) Topoisomerase poisons: Harnessing the dark side of enzyme mechanism. J Biol Chem. 270: 21429.
(3) Chen AY, Liu LF (1994) DNA topoisomerases: Essential enzymes and lethal targets. Annu Rev Pharma Toxicol. 34: 191.
(4) Tewey KM; Rowe TC, Yang L, Halligan BD, Liu LF (1984) Adriamycin-induced DNA damage mediated by mammalian DNA topoisomerase II. Science 226: 466.
(5) Liu LF (1989) DNA topoisomerase poisons as antitumor drugs. Annu Rev Biochem. 58: 351.
(6) Pommier Y, Kohn K (1989) Topoisomerase II inhibition by antitumor intercalators and demethylepipophyllatoxins. In: R I Glazer (ed) Development in Cancer Chemotherapy. Boca Raton FL: CRC Press Inc. p 175.
(7) Jensen PB, Serensen BS, Sehested M, Grue P, Demant EJF, Hansen HH (1994) Targeting the cytotoxicity of topoisomerase II directed epipodophyllotoxins to tumor cells in acidic environments. Cancer res. 54: 2959.
(8) Sehested M, Jensen PB. (1996) Mapping of DNA topoisomerase II poisons (etoposide, derocidin) and catalytic inhibitors (aclarubicin, ICRF-187) to four distinct steps in the topoisomerase II catalytic cycle. Biochem Pharmacol. 51: 879.
(9) Sørensen BS, Sinding J, Andersen AH, Alsner J, Jensen PB, Westergaard O (1992) Mode of action of topoisomerase II targeting agents at a specific DNA sequence: Uncoupling the DNA binding, cleavage and religation events. J Mol Biol. 228: 778.
(10) Tanabe K, Ikegami Y, Ishida R, Andoh T (1991) Inhibition of topoisomerase II by antitumor agents bis(2,6-dioxopiperazine) derivatives. Cancer Res. 51: 4903.
(11) Berger JM, Gamblin 5J, Harrison SC, Wang JC (1996) Structure and mechanism of DNA topoisomerase II. Nature 379: 225.
(12) Roca J. Ishida R, Berger JM, Andoh T, Wang JC (1994) Antitumour bisdioxopiperazines inhibit yeast DNA topoisomerase II by trapping the enzyme in the form of a closed protein clamp. Proc Natl Acad Sci USA, 91: 1781.
(13) Roca J, Berger JM, Harrison SC, Wang JC (1996) DNA transport by a type II topoisomerase: Direct evidence for a two-gate mechanism. Proc Natl Acad Sci USA 93: 4057.
(14) Roca J, Wang JC (1994) DNA transport by a type II DNA topoisomerase: Evidence in favor of a two-gate mechanism. Cell, 77: 609.
(15) Sehested M, Jensen PB, Sørensen BS, Holm B, Friche E, Demant EJF (1993) Antagonistic effect of the cardioprotector (+)-1,2-bis(3,5-dioxopiperazinyl-1-yl)propane (ICRF-187) on DNA breaks and cytotoxicity induced by the topoisomerase II directed drug daunorubicin and etoposide (VP-16). Biochem Pharmacol. 46: 389.
(16) Hasinoff BB (1990) The iron(III) and copper(II) complexes of adriamycin promote the hydrolysis of the cardioprotective agent ICRF-187 ((+)-1,2-bis(3,5-dioxopiperazinyl-1-yl)propane). Agents and Actions 29: 374.
(17) Sehested M, Wessel I, Jensen LH, Holm B, Olivieri RS, Kenwrick S, Creighton AM, Nitiss JL, Jensen PB (1998) Chinese hamster ovary cells resistant to the topoisomerase II catalytic inhibitor ICRF-159; a Tyr49Phe mutation confers high level resistance to bisdioxopiperazines. Cancer Res. 58: 1460.
(18) Wessel I, Jensen LH, Jensen PB, Falck 3, Roerth M, Nitiss JL, Sehested M (1998) Human small cell lung cancer NYH cells selected for resistance to the bisdioxopiperazine topoisomerase II (topoII) catalytic inhibitor ICRF-187 (NYH/187) demonstrate a Functional Arg162Gln mutation in the walker A consensus ATP binding site of the a isoform. [abstract] Proc AACR 39: 375.
(19) Langer SW, Sehested M, Jensen P8 (2000) Treatment of anthracycline extravasation with dexrazoxane. Clin Cancer Res. 6(9): 3680.
(20) Langer SW, Sehested M, Jensen PB (2001) Dexrazoxane is a potent and specific inhibitor of anthracycline induced subcutaneous lesions in mice. Annals of Oncology, *in print*.
(21) Langer SW, Buter J, Glaccone G, Sehested M, Jensen PB (2000) Dexrazoxane in Anthracycline Extravasation. J Clin Oncol. 18(16): 3064.
(22) Jensen PB, Sehested M (1997) DNA Topoisomerase II Rescue by Catalytic Inhibitors. Biochem Pharmacol, 54: 755.
(23) Kohn (1981) In DNA Repair: A Laboratory Manual of Research Procedures; Fridberg EC, Hanawalt HC Eds. Marcel Dekker Inc. New York, 379-401.
(24) Sahai BM, Kaplan JG (1986) A quantitative decatenation assay for type II topoisomerases. Anal Blochem 156: 364.
(25) Szmigiero L, Studzian K (1988) H₂O₂ as a DNA fragmenting agent in the alkaline elution interstrand crosslinking and DNA-protein crosslinking assays. Analyt Biochem 168: 88.
(26) Wessel I, Jensen LH, Jensen PB, Falck J, Rose A. Roerth M, Nitiss JL, Sehested M (1999) Human Small Cell Lung Cancer NYH Cells Selected for Resistance to the Bisdioxopiperazine Topoisomerase II Catalytic Inhibitor ICRF-187 Demonstrate a Functional R162Q Mutation in the Walker A Consensus ATP Binding Domain of the α Isoform. Cancer Res. 59: 3442.
(27) Zwelling LA, Hinds M, Chan D, Mayes J, Sie KL, Parker E, Silberman L, Radcliffe A, Beran M, Blick M (1989) Characterization of an amsacrine-reslstant line of human leukemia cells. Evidence for a drug-resistant form of topoisomerase II. J Biol Chem 264: 16411.
(28) Wang et al, Biochemistry (2001); 40; 3316-3323
(29) Burden DA, Froelich-Ammon SJ, and Osheroff N (2001) Topoisomerase II-mediated cleavage of plasmid DNA. In methods in molecular biology vol. 95, DNA topoisomerase protocols enzymology and drugs pp 283- 289. Humana Press, Editors Osheroff N and Bjornsti MA, ISBN 0-89603-512-3 .

## Claims

1. Use of a compound of formula M, quaternary ammonium salts thereof, or compositions comprising either entity, for the preparation of a medicament for the treatment or prevention of extravasation, 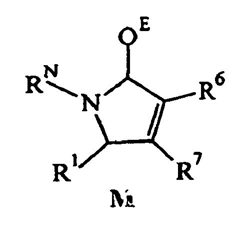
-O^{E} and R¹ is a carbonyl equivalent independently selected from the group consisting of =O, =S; -OR², -SR², dithiane, dioxane and dioxolane;
R^{N} is selected from the group consisting of hydrogen, optionally substituted C₁₋₆-alkyl, optionally substituted C₂₋₁₀-alkenyl, optionally substituted C₂₋₁₀-alkynyl, optionally substituted aryl, optionally substituted heteroaryl, optionally substituted heterocycle, optionally substituted C₃₋C₇-cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³), C(C=S)-N(R³)(R³);
wherein at least one of R⁶ and R⁷ are independently selected from group consisting of hydrogen, halogen, hydroxy, primary, secondary or tertiary amine, optionally substituted C₁₋₄-alkyl, optionally substituted C₂₋₅-alkenyl, optionally substituted C₂₋₆-alkynyl, optionally substituted aryl, optionally substituted heteroaryl, optionally substituted heterocycle, and optionally substituted C₃-C₇ cycloalkyl;
and the other of R⁶ and R⁷ is A-Y-Z, wherein
-A- is selected from the group consisting of hydrogen, -C(R²)(R²)-, -C(=O)-, -N(R^{N})-, -O-, -S-,-P-, -P(O)-;
Y is a biradical which may be absent or independently selected from one of the group consisting of optionally substituted C₁₋₆-alkyl, optionally substituted C(=O)-C₁₋₆₋alkyl, optionally substituted C₁₋₆-alkyl-C(=O), optionally substituted C₂₋₁₀-alkenyl, optionally substituted C₂₋₁₀₋alkynyl, optionally substituted C₃₋₈-carbocycle and optionally substituted heterocycle;
Z is a monoradical independently selected from the group consisting of optionally substituted aryl, optionally substituted heteroaryl, optionally substituted C₃₋₈-carbocyle, optionally substituted heterocycle, H, OR^{Z}, N(R^{Z})(R³), S-R^{z}, NO₂, -CN, and halogen;
wherein R^{Z} is selected from the group consisting of hydrogen, optionally substituted C₁₋₄ alkyl, optionally substituted C₂₋₅ alkenyl, optionally substituted C₂₋₆ alkynyl, optionally substituted aryl, optionally substituted heteroaryl, optionally substituted heterocycle, and optionally substituted C₃-C₇ cycloalkyl;
the term "optionally substituted" meaning that the group in question may be substituted 1 to 5 times with one or more groups selected from C₁₋₆-alkyl, C₁₋₆-alkoxyl, oxo (which may be represented in the tautomeric enol form), carboxyl, amino, hydroxyl (which when present in an enol system may be represented in the tautomeric keto form), nitro, sulphono, sulphanyl, sulfoxide, C₁₋₆-carboxyl, C₁₋₆-alkoxycarbonyl, C₁₋₆-alkylcarbonyl, formyl; aryl, aryloxy, aryloxycarbonyl, arylcarbonyl, heteroaryl, amino, mono- and di(C₁₋₆-alkyl)amino; carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, cyano, guanidino, carbamido, C₁₋₆-alkanoyloxy, C₁₋₆-alkylsulphonyloxy, dihalogen-C₁₋₆-alkyl, trihalogen-C₁₋₆-alkyl, and halogen.

2. The use according to claim 1, wherein O^{E} and R¹ are =O.

3. The use according to any one of the preceding claims, wherein A is selected from the group consisting of C(R²R³), N(R²), O, and S, preferably N(R²), O, and S, most preferably N(R²) and S.

4. The use according to any one of the preceding claims, wherein Y is selected from the group consisting of C₁₋₆-alkyl, C₂₋₁₀-alkenyl, C₂₋₁₀-alkynyl, each of which may be optionally substituted, preferably wherein Y is optionally substituted C₁₋₆-alkyl.

5. The use according to any one of the preceding claims, wherein Z is selected from the group consisting of hydrogen, optionally substituted aryl, optionally substituted heteroaryl, optionally substituted C₃₋₈-carbocyle, optionally substituted heterocycle, OR^{Z}, N(R^{Z})(R³), S-R^{Z}, preferably wherein Z is selected from the group consisting of hydrogen, optionally substituted heteroaryl, optionally substituted heterocycle and N(R^{Z})(R³), most preferably Z is selected from the group consisting of hydrogen, optionally substituted heterocycle and N(R^{Z})(R³).

6. The use according to any one of the preceding claims, wherein one of R⁶ and R⁷ is hydrogen and the other of R⁶ and R⁷ is A-Y-Z, wherein Z is selected from the group consisting of N(R⁸)(R⁹) and optionally substituted heteroaryl.

7. The use according to any one of claims 5 to 6, wherein the optionally substituted heterocycle is selected from the group consisting of succinimide, imidazole, pyrazole, pyrrole, oxazole, furazan, barbituric acid, thiobarbituric acid, dioxopiperazine, hydantoin, dihydrouracii, and 3-alkoxylsoxazole, each of which may be optionally substituted, preferably wherein the heterocycle is selected from the group consisting of succinimide, imidazole, pyrazole, pyrrole, oxazole, and furazan, each of which may be optionally substituted.

8. The use according to any one of claims 3 to 7, wherein R¹ is =O and at least one of R⁶ and R⁷ Is hydrogen.

9. The use according to claim 1, wherein R⁶ and R⁷ may together form a ring selected from the group consisting of C₃₋₈-carbocycle, heterocycyl, aryl or heteroaryl, each of which may optionally be substituted.

10. The use according to claim 8, wherein R⁶ and R⁷ may together form a C₃₋₈-carbocycle, such as cyclohexane.

11. The use according to claim 8, wherein O^{E} is =O, R¹ is =O, R^{N} is hydrogen, one of R⁶ and R⁷ is hydrogen, and the other of R⁶ and R⁷ is C₁₋₆-alkyl.

12. The use according to claim 1, wherein one of R⁶ and R⁷ is hydrogen, and the other of R⁶ and R⁷ is selected from methyl or ethyl.

13. The use according to claim 1, wherein compound of formula M is compound TT0043, wherein O^{E} is =O, R¹ is =O, R^{N} is hydrogen, one of R⁶ and R⁷ is hydrogen, and the other of R⁶ and R⁷ is methyl.

14. The use according to claim 1 to 2, wherein one of R⁶ and R⁷ is hydrogen and the other of R⁶ and R⁷ is A-Y-Z , wherein A is selected from the group consisting of hydrogen, -C(R²)(R²)-,-C(=O)-, -N(R^{N})-, -O-, -S-, -P-, -P(O)-; Y may be absent or selected from one of the group consisting of optionally substituted C₁₋₆-alkyl, optionally substituted C(=O)-C₁₋₆-alkyl, optionally substituted C₁₋₆-alkyl-C(=O), optionally substituted C₂₋₁₀-alkenyl, optionally substituted C₂₋₁₀₋alkynyl, optionally substituted C₃-₈-carbocycle and optionally substituted heterocycle; and Z is a monoradical selected from the group consisting of optionally substituted aryl, optionally substituted heteroaryl, optionally substituted C₃₋₈-carbocyle, optionally substituted heterocycle, H, OR^{Z}, N(R^{Z})(R³), S-R^{Z}, NO₂, -CN, and halogen.

15. The use according to claim 14, wherein one of R⁶ and R⁷ is hydrogen and the other of R⁶ and R⁷ is A-Y-Z wherein A is selected from the group consisting of hydrogen, -C(R²)(R²)-,-C(=O)-, -N(R^{N})-, -O-, -S-, -P-, -P(O)-; Y may be absent or selected from one of the group consisting of optionally substituted C₁₋₆-alkyl, optionally substituted C(=O)-C₁₋₆-alkyl, optionally substituted C₁₋₆-alkyl-C(=O), optionally substituted C₂₋₁₀-alkenyl, optionally substituted C₂₋₁₀₋alkynyl, optionally substituted C₃₋₈-carbocycle and optionally substituted heterocycle; and wherein Z is selected from the group consisting of N(R⁸)(R⁹), optionally substituted heteroaryl and optionally substituted heterocycle.

16. The use according to claim 15, wherein Z is N(R⁸)(R⁹), wherein R⁸ and R⁹ may independently be selected from the group consisting of hydrogen, optionally substituted C₁₋₆₋alkyl, optionally substituted C₂₋₁₀-alkenyl, optionally substituted C₂₋₁₀-alkynyl, optionally substituted aryl, optionally substituted heteroaryl, optionally substituted heterocycle, optionally substituted C₃-C₇-cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³) and C(C=S)-N(R³)(R³), preferably wherein at least one of R⁸ and R⁹ is selected from the group consisting of optionally substituted heteroaryl and optionally substituted heterocycle, most preferably optionally substituted heterocycle.

17. The use according to claim 1, wherein the compound of formula M is selected from the group consisting of maleimide, N-methyl-maleimide (NMM) and N-ethyl-maleimide (NEM).

18. The use according to claim 1, wherein compound of formula M is compound TT006,

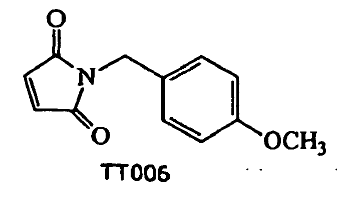

19. The use according to claim 1, wherein compound of formula M is compound TT0046,

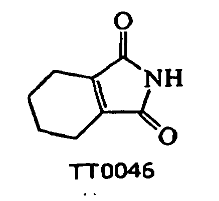

20. The use according to claim 1, wherein compound of formula M is compound TT0048,

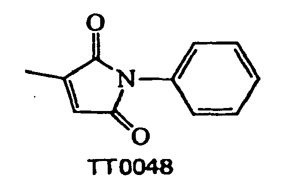

21. The use according to claim 1, wherein compound of formula M is compound TT0051,

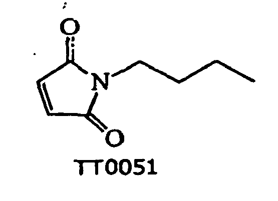

22. The use according to claim 1, wherein compound of formula M is a compound selected from the group consisting of M-i, M-ii, M-iii, M-iv, M-v, M-vi and M-vii 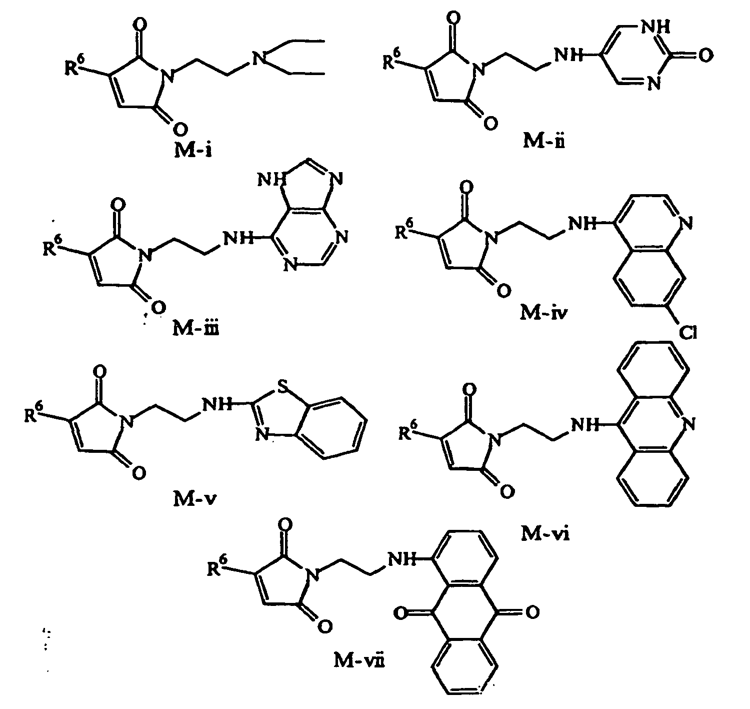
wherein R⁶ is A-Y-Z , wherein A is selected from the group consisting of hydrogen, -C(R²)(R²)-, -C(=O)-, -N(R^{N})-, -O-, -S-, -P-, -P(O)-; Y may be absent or selected from one of the group consisting of optionally substituted C₁₋₆-alkyl, optionally substituted C(=O)-C₁₋₆-alkyl, optionally substituted C₁₋₆-alkyl-C(=O), optionally substituted C₂₋₁₀-alkenyl, optionally substituted C₂₋₁₀₋alkynyl, optionally substituted C₃₋₈-carbocycle and optionally substituted heterocycle; and Z is a monoradical selected from the group consisting of optionally substituted aryl, optionally substituted heteroaryl, optionally substituted C₃₋₈-carbocyle, optionally substituted heterocycle, H, OR^{Z}, N(R^{Z})(R³), S-R^{Z}, NO₂, -CN, and halogen.

23. The use according to claim 1, wherein compound of formula M is a compound selected from the group consisting of M-viii, M-ix, M-x and M-xi 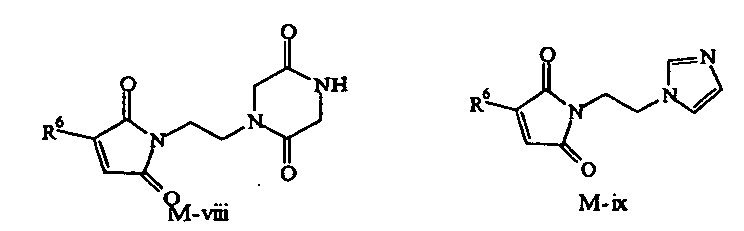 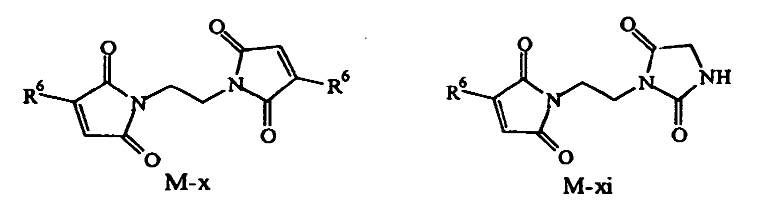
wherein R⁶ is A-Y-Z , wherein A is selected from the group consisting of hydrogen, -C(R²)(R²)-, -C(=O)-, -N(R^{N})-, -O-, -S-, -P-, -P(O)-; Y may be absent or selected from one of the group consisting of optionally substituted C₁₋₆-alkyl, optionally substituted C(=O)-C₁₋₆-alkyl, optionally substituted C₁₋₆-alkyl-C(=O), optionally substituted C₂₋₁₀-alkenyl, optionally substituted C₂₋₁₀₋alkynyl, optionally substituted C₃₋₈-carbocycle and optionally substituted heterocycle; and Z is a monoradical selected from the group consisting of optionally substituted aryl, optionally substituted heteroaryl, optionally substituted C₃₋₈-carbocyle, optionally substituted heterocycle, H, OR^{Z}, N(R^{Z})(R³), S-R^{Z}, NO₂, -CN, and halogen.

24. The use according to claim 1, wherein compound of formula M is a compound of formula M-xii 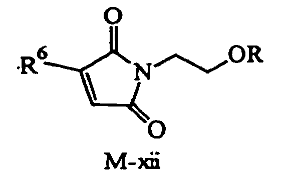
wherein OR is selected from an alcohol, an ether and an ester.

25. The use according to claim 1, wherein compound of formula M is a compound of formula M-xiii 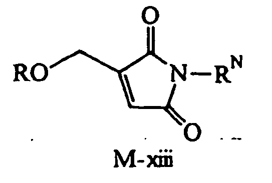
wherein OR is selected from an alcohol, an ether and an ester and
R^{N} is selected from the group consisting of hydrogen, optionally substituted C₁₋₆-alkyl, optionally substituted C₂₋₁₀-alkenyl, optionally substituted C₂₋₁₀-alkynyl, optionally substituted aryl, optionally substituted heteroaryl, optionally substituted heterocycle, optionally substituted C₃₋C₇-cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³), C(C=S)-N(R³)(R³).

26. Use according to any of claims 1 to 25, wherein the extravasation is due to a topoisomerase II poison.

27. Use of a compound of formula M as defined in any of claims 1 to 26, quaternary ammonium salts thereof, or compositions comprising either entity, for the preparation of a medicament for the treatment of cancer.

28. Use of a combination of a topoisomerase II poison and a compound of formula M as defined in any of claims 1 to 26, quaternary ammonium salts thereof, or compositions comprising either entity, for the preparation of a medicament for the treatment of cancer.

29. Use according to claim 28, wherein said topoisomerase II poison is selected from the group consisting of doxorubicin, daunorubicin, dactinomycin, epirubicin, bisantrene, amsacrine, mitomycin C, vincristine, vinblastine, vindesine, liposomal anthracyclines, mitoxantrone, esorubicin, menogaril, acalcinomycin, cisplatin, fluorouracil, etoposide and bleomycin.

30. A pharmaceutical composition comprising at least one compound selected from the group consisting of formula M, as defined in any of the preceding claims, and one or more chemotherapeutic agents selected from the group consisting of doxorubicin, daunorubicin, dactinomycin, epirubicin, bisantrene, amsacrine, mitomycin C, vincristine, vinblastine, vindesine, liposomal anthracyclines, mitoxantrone, esorubicin, menogaril, acalcinomycin, dsplatin, fluorouracil, etoposide and bleomycin, and at least one pharmaceutically acceptable excipient or carrier.

31. A composition according to claim 30, wherein the compound of formula M is of the formula maleimide, NMM, NEM, TT006, TT0043, TT0046, TT0048, TT0051, M-i, M-ii, M-iii, M-iv, M-v, M-vi, M-vii, M-viii, M-ix, M-x, M-xi, M-xii, M-xiii, as defined in claims 13, and 17-25.

## Revendications

1. Usage d'un composé de formule M, de sels d'ammonium quaternaires de celui-ci, ou de compositions comprenant quelqu'une de ces entités, pour la préparation d'un médicament pour le traitement ou la prévention de l'extravasation, 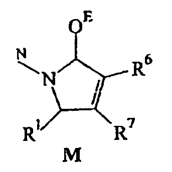
-O^{E} et R¹ sont des équivalents carbonyles choisis indépendamment dans le groupe consistué par =O, =S; -OR², -SR², le dithiane, le dioxane et le dioxolane ;
R^{N} est choisi dans le groupe consistué par l'hydrogène, un alkyle C₁₋₆ facultativement substitué, un alkenyle C₂₋₁₀ facultativement substitué, un alkynyle C₂₋₁₀ facultativement substitué, un aryle facultativement substitué, un hétéroaryle facultativement substitué, un hétérocycle facultativement substitué, un cycloalkyle C₃-C₇ facultativement substitué, CH₂₋N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³), C(C=S)-N(R³)(R³) ;
où au moins l'un de R⁶ et R⁷ est choisi indépendamment dans le groupe consistué par l'hydrogène, un halogène, une hydroxyamine, une amine primaire, secondaire ou tertiaire, un alkyle C₁₋₄ facultativement substitué, un alkenyle C₂₋₅ facultativement substitué, un alkynyle C₂₋₆ facultativement substitué, un aryle facultativement substitué, un hétéroaryle facultativement substitué, un hétérocycle facultativement substitué, et un cycloalkyle C₃-C₇ facultativement substitué ;
et l'autre de R⁶ et R⁷ est A-Y-Z, où
-A- est choisi dans le groupe consistué par l'hydrogène, -C(R²)(R²)-, -C(=O)-, -N(R^{N})-, -O-, -S-, -P-, -P(O)-;
Y est un biradical pouvant être absent ou choisi indépendamment parmi les éléments du groupe consistué par un alkyle C₁₋₆ facultativement substitué, un C(=O)-C₁₋₆-alkyle facultativement substitué, un C₁₋₆-alkyl-C(=O) facultativement substitué, un alkenyle C₂₋₁₀ facultativement substitué, un alkynyle C₂₋₁₀ facultativement substitué, un carbocycle C₃₋₈ facultativement substitué et un hétérocycle facultativement substitué ;
Z est un monoradical choisi indépendamment dans le groupe consistué par un aryle facultativement substitué, un hétéroaryle facultativement substitué, un carbocycle C₃₋₈ facultativement substitué, un hétérocycle facultativement substitué, H, OR^{Z}, N(R^{Z})(R³), S-R^{Z}, NO₂, -CN, et un halogène ;
où R^{Z} est choisi dans le groupe consistué par l'hydrogène, un alkyle C₁₋₄ facultativement substitué, un alkenyle C₂₋₅ facultativement substitué, un alkynyle C₂₋₆ facultativement substitué, un aryle facultativement substitué, un hétéroaryle facultativement substitué, un hétérocycle facultativement substitué, et un cycloalkyle C₃-C₇ facultativement substitué ;
le terme "facultativement substitué" signifiant que le groupe en question peut être substitué 1 à 5 fois avec un ou plusieurs groupes choisis parmi les groupes C₁₋₆ alkyle, C₁₋₆ alkoxyle, oxo (qui peut être représenté par la forme tautomérique énol), carboxyle, amino, hydroxyl (qui peut lorsque présent dans un système énol être représenté par la forme tautomérique céto), nitro, sulfono, sulfanyle, sulfoxide, C₁₋₆ carboxyle, C₁₋₆ alkoxycarbonyle, C₁₋₆ alkylcarbonyle, formyle, aryle, aryloxy, aryloxycarbonyle, arylcarbonyle, hétéroaryle, amino, mono- et di(C₁₋₆-alkyl)amino ; carbamoyle, mono- et di(C₁₋₆-alkyl)aminocarbonyle, amino-C₁₋₆-alkyl-aminocarbonyle, mono- et di(C₁₋₆-alkyl)amino-C₁₋₆-alkyl-aminocarbonyle, C₁₋₆₋alkylcarbonylamino, cyano, guanidino, carbamido, C₁₋₆-alkanoyloxy, C₁₋₆-alkylsulphonyloxy, dihalogéno-C₁₋₆-alkyle, trihalogéno-C₁₋₆-alkyle, et halogène.

2. L'usage selon la revendication 1, où O^{E} et R¹ sont =O.

3. L'usage selon une quelconque des revendications précédentes, où A est choisi dans le groupe consistué par C(R²R³), N(R²), O, et S, de préférence N(R²), O, et S, au mieux N(R²) et S.

4. L'usage selon une quelconque des revendications précédentes, où Y est choisi dans le groupe consistué par un alkyle C₁₋₆, un alkenyle C₂₋₁₀, un alkynyle C₂₋₁₀, chacun de ceux-ci pouvant être facultativement substitué, de préférence où Y est un alkyle C₁₋₆ substitué.

5. L'usage selon une quelconque des revendications précédentes, où Z est choisi dans le groupe consistué par l'hydrogène, un aryle facultativement substitué, un hétéroaryle facultativement substitué, un carbocycle C₃₋₈ facultativement substitué, un hétérocycle facultativement substitué, OR^{Z}, N(R^{Z})(R³), S-R^{Z}, de préférence où Z est choisi dans le groupe consistué par l'hydrogène, un hétéroaryle facultativement substitué, un hétérocycle facultativement substitué et N(R^{Z})(R³), au mieux Z est choisi dans le groupe consistué par l'hydrogène, un hétérocycle facultativement substitué et N(R^{Z})(R³).

6. L'usage selon une quelconque des revendications précédentes, où l'un de R⁶ ou R⁷ est l'hydrogène et l'autre de R⁶ ou R⁷ est A-Y-Z, où Z est choisi dans le groupe consistué par N(R⁸)(R⁹) et un hétéroaryle facultativement substitué.

7. L'usage selon une quelconque des revendications 5 à 6, où l'hétérocycle facultativement substitué est choisi dans le groupe consistué par le succinimide, l'imidazole, le pyrazole, le pyrrole, l'oxazole, le furazane, l'acide barbiturique, l'acide thiobarbiturique, la dioxopipérazine, l'hydantoine, le dihydrouracile, et l'alkoxy-3-isoxazole, chacun de ceux-ci pouvant être facultativement substitué, de préférence où l'hétérocycle est choisi dans le groupe consistué par le succinimide, l'imidazole, le pyrazole, le pyrrole, l'oxazole, et le furazane, chacun de ceux-cl pouvant être facultativement substitué.

8. L'usage selon une quelconque des revendications 3 à 7, où R¹ est =O et au moins l'un de R⁶ et R⁷ est l'hydrogène.

9. L'usage selon la revendication 1, où R⁶ et R⁷ peuvent ensemble former un cycle choisi dans le groupe consistué par un carbocycle C₃₋₈, un hétérocycle, un aryle ou un hétéroaryle, chacun de ceux-ci pouvant être facultativement substitué.

10. L'usage selon la revendication 8, où R⁶ et R⁷ peuvent ensemble former un carbocycle C₃₋₈, tel que le cyclohexane.

11. L'usage selon la revendication 8, où O^{E} est =0, R¹ est =O, R^{N} est l'hydrogène, l'un de R⁶ et R⁷ est l'hydrogène, et l'autre de R⁶ et R⁷ est un alkyle C₁₋₆.

12. L'usage selon la revendication 1, où l'un de R⁶ et R⁷ est l'hydrogène, et l'autre de R⁶ et R⁷ est choisi parmi le méthyle ou l'éthyle.

13. L'usage selon la revendication 1, où le composé de formule M est le composé TT0043, dans laquelle O^{E} est =O, R¹ est =O, R^{N} est l'hydrogène, l'un de R⁶ et R⁷ est l'hydrogène, et l'autre de R⁶ et R⁷ est le méthyle.

14. L'usage selon les revendications 1 à 2, où l'un de R⁶ et R⁷ est l'hydrogène et l'autre de R⁶ et R⁷ est A-Y-Z, où A est choisi dans le groupe consistué par l'hydrogène, -C(R²)(R²)-, -C(=O)-, -N(R^{N})-, -O-, -S-, -P-, -P(O)- ; Y peut être absent ou choisi parmi les éléments du groupe consistué par un alkyle C₁₋₆ facultativement substitué, un C(=O)-C₁₋₆-alkyle facultativement substitué, un C₁₋₆-alkyl-C(=O) facultativement substitué, un alkenyle C₂₋₁₀ facultativement substitué, un alkynyle C₂₋₁₀ facultativement substitué,un carbocycle C₃₋₈ facultativement substitué et un hétérocycle facultativement substitué ; et Z est un monoradical choisi dans le groupe consistué par un aryle facultativement substitué, un hétéroaryle facultativement substitué, un carbocycle C₃₋₈ facultativement substitué, un hétérocycle facultativement substitué, H, OR^{Z}, N(R^{Z})(R³), S-R^{Z}, NO₂, -CN, et un halogène.

15. L'usage selon la revendication 14, où l'un de R⁶ et R⁷ est l'hydrogène et l'autre de R⁶ et R⁷ est A-Y-Z où A est choisi dans le groupe consistué par l'hydrogène, -C(R²)(R²)-, -C(=O)-, -N(R")-, -O-, -S-, -P-, -P(O)- ; Y peut être absent ou choisi parmi les éléments du groupe consistué par un alkyle C₁₋₆ facultativement substitué, un C(=O)-C₁₋₆-alkyle facultativement substitué, un C₁₋₆-alkyl-C(=O) facultativement substitué, un alkenyle C₂₋₁₀ facultativement substitué, un alkynyle C₂₋₁₀ facultativement substitué, un carbocycle C₃₋₈ facultativement substitué et un hétérocycle facultativement substitué ; et où Z est choisi dans le groupe consistué par N(R⁸)(R⁹), un hétéroaryle facultativement substitué et un hétérocycle facultativement substitué.

16. L'usage selon la revendication 15, où Z est N(R⁸)(R⁹), où R⁸ et R⁹ peuvent être choisis indépendamment dans le groupe consistué par l'hydrogène, un alkyle C₁₋₆ facultativement substitué, un alkenyle C₂₋₁₀ facultativement substitué, un alkynyle C₂₋₁₀ facultativement substitué, un aryle facultativement substitué, un hétéroaryle facultativement substitué, un hétérocycle facultativement substitué, un cycloalkyle C₃-C₇ facultativement substitué, CH₂₋N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³) et C(C=S)-N(R³)(R³), de préférence où au moins l'un de R⁸ et R⁹ est choisi dans le groupe consistué par un hétéroaryle facultativement substitué et un hétérocycle facultativement substitué, au mieux un hétérocycle facultativement substitué.

17. L'usage selon la revendication 1, où le composé de formule M est choisi dans le groupe consistué par le malelmide, le N-méthyl-maleimide (NMM) et le N-éthyl-maleimide (NEM).

18. L'usage selon la revendication 1, où le composé de formule M est le composé TT006,

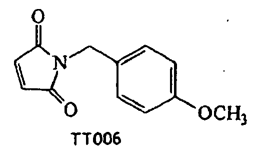

19. L'usage selon la revendication 1, où le composé de formule M est le composé TT0046,

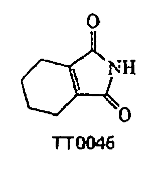

20. L'usage selon la revendication 1, où le composé de formule M est le composé TT0048,

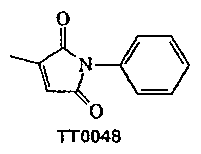

21. L'usage selon la revendication 1, où le composé de formule M est le composé TT0051,

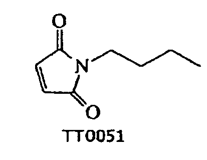

22. L'usage selon la revendication 1, où le composé de formule M est un composé choisi dans le groupe consistué par M-i, M-ii, M-iii, M-iv, M-v, M-vi et M-vii 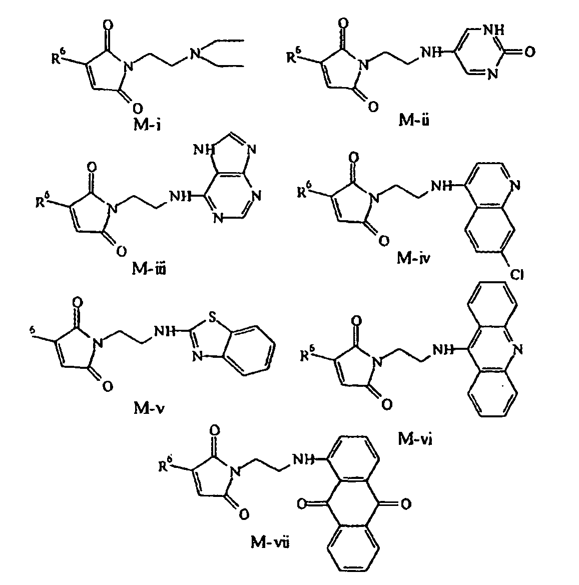
dans laquelle R⁶ est A-Y-Z, où A est choisi dans le groupe consistué par l'hydrogène, -C(R²)(R²)-, -C(=O)-, -N(R^{N})-, -O-, -S-, -P-, -P(O)- ; Y peut être absent ou choisi parmi les éléments du groupe consistué par un alkyle C₁₋₆ facultativement substitué, un C(=O)-C₁₋₆₋alkyle facultativement substitué, un C₁₋₆-alkyl-C(=O) facultativement substitué, un alkenyle C₂₋₁₀ facultativement substitué, un alkynyle C₂₋₁₀ facultativement substitué, un carbocycle C₃₋₈ facultativement substitué et un hétérocycle facultativement substitué ; et Z est un monoradical choisi dans le groupe consistué par un aryle facultativement substitué, un hétéroaryle facultativement substitué, un carbocycle C₃₋₈ facultativement substitué, un hétérocycle facultativement substitué, H, OR², N(R²)(R³), S-R², NO₂, -CN, et un halogène.

23. L'usage selon la revendication 1, où le composé de formule M est un composé choisi dans le groupe consistué par M-viii, M-ix, M-x et M-xi 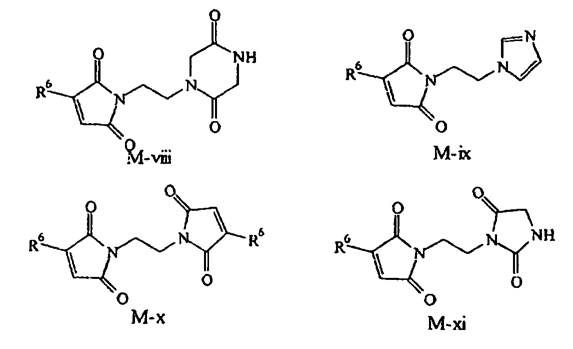
dans laquelle R⁶ est A-Y-Z, où A est choisi dans le groupe consistué par l'hydrogène, -C(R²)(R²)-, -C(=O)-, -N(R^{N})-, -O-, -S-, -P-, -P(O)- ; Y peut être absent ou choisi parmi les éléments du groupe consistué par un alkyle C₁₋₆ facultativement substitué, un C(=O)-C₁₋₆₋alkyle facultativement substitué, un C₁₋₆-alkyl-C(=O) facultativement substitué, un alkenyle C₂₋₁₀ facultativement substitué, un alkynyle C₂₋₁₀ facultativement substitué, un carbocycle C₃₋₈ facultativement substitué et un hétérocycle facultativement substitué ; et Z est un monoradical choisi dans le groupe consistué par un aryle facultativement substitué, un hétéroaryle facultativement substitué, un carbocycle C₃₋₈ facultativement substitué, un hétérocycle facultativement substitué, H, OR^{Z}, N(R^{Z})(R³), S-R^{Z}, NO₂, -CN, et un halogène.

24. L'usage selon la revendication 1, où le composé de formule M est un composé de 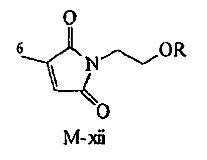
formule M-xii
dans laquelle OR est choisi parmi un alcool, un éther et un ester.

25. L'usage selon la revendication 1, où le composé de formule M est un composé de formule M-xiii 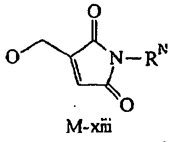
dans laquelle OR est choisi parmi un alcool, un éther et un ester et
R^{N} est choisi dans le groupe consistué par l'hydrogène, un alkyle C₁₋₆ facultativement substitué, un alkenyle C₂₋₁₀ facultativement substitué, un alkynyle C₂₋₁₀ facultativement substitué, un aryle facultativement substitué, un hétéroaryle facultativement substitué, un hétérocycle facultativement substitué, un cycloalkyle C₃-C₇ facultativement substitué, CH₂-N(R³)(R³), CH₂-OR³, CH₂-SR³, CH₂-O-C(=O)R³, CH₂-O-C(=O)-OR³, CH₂-O-C(=S)R³, CH₂₋S-C(=O)R³, C(=O)(R³), C(=S)R³, -C(=S)-OR³, -C(=O)-SR³, C(=O)-N(R³)(R³), C(C=S)-N(R³)(R³).

26. L'usage selon une quelconque des revendications 1 à 25, où l'extravasation est due à un poison de la topoisomérase II.

27. Usage d'un composé de formule M telle que définie dans une quelconque des revendications 1 à 26, des sels d'ammonium quaternaires de celui-ci, ou de compositions comprenant quelqu'une de ces entités, pour la préparation d'un médicament pour le traitement du cancer.

28. Usage d'une combinaison d'un poison de la topoisomérase II et d'un composé de formule M telle que définie dans une quelconque des revendications 1 à 26, des sels d'ammonium quaternaires de celui-ci, ou de compositions comprenant quelqu'une de ces entités, pour la préparation d'un médicament pour le traitement du cancer.

29. Usage selon la revendication 28, où ledit poison de la topoisomérase II est choisi dans le groupe consistué par la doxorubicine, la daunorubicine, la dactinomycine, l'épirublcine, le bisantrène, l'amsacrine, la mitomycine C, la vincristine, la vinblastine, la vindésine, les anthracyclines liposomales, la mitoxantrone, l'ésorubicine, le ménogaril, l'acalcinomycine, le cisplatine, le fluorouracile, l'étoposide et la bléomycine.

30. Composition pharmaceutique comprenant au moins un composé choisi dans le groupe consistué par les formules M, telles que définies dans une quelconque des revendications précédentes, et un ou plusieurs agents chimiothérapeutiques choisi dans le groupe consistué par la doxorubicine, la daunorubicine, la dactinomycine, l'épirubicine, le bisantrène, l'amsacrine, la mitomycine C, la vincristine, la vinblastine, la vindésine, les anthracyclines liposomales, la mitoxantrone, l'ésorubicine, le ménogaril, l'acalcinomycine, le cisplatine, le fluorouracile, l'étoposide et la bléomycine, et au moins un excipient ou porteur acceptable pharmaceutiquement.

31. Composition selon la revendication 30, dans laquelle le composé de formule M est de la formule maleimide, NMM, NEM, TT006, TT0043, TT0046, TT0048, TT0051, M-i, M-ii, M-iii, M-iv, M-v, M-vi, M-vii, M-vill, M-ix, M-x, M-xi, M-xii, M-xiii, telles que définies dans les revendications 13, et 17-25.

## Patentansprüche

1. Anwendung einer Verbindung der Formel M, quartärer Ammoniumsalze hiervon oder Zusammensetzungen umfassend eine der Einheiten zur Herstellung eines Arzneimittels für die Behandlung oder Vorbeugung von Extravasation, 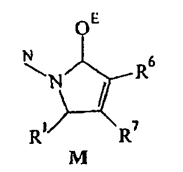
-O^{E} und R¹ sind ein Carbonyläquivalent, das unabhängig ausgewählt ist aus der Gruppe bestehend aus =O, =S; -OR², -SR², Dithian, Dioxan und Dioxolan;
R^{N} ist ausgewählt aus der Gruppe bestehend aus Hydrogen, gegebenenfalls substituiertem C₁₋₆-Alkyl, gegebenenfalls substituiertem C₂₋₁₀-Alkenyl, gegebenenfalls substituiertem C₂₋₁₀-Alkynyl, gegebenenfalls substituiertem Aryl, gegebenenfalls substituiertem Heteroaryl, gegebenenfalls substituiertem Heterocyclus, gegebenenfalls substituiertem C₃-C₇-Cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³), C(C=S)-N(R³)(R³);
wo mindestens eins von R⁶ und R⁷ unabhängig ausgewählt ist aus der Gruppe bestehend aus Hydrogen, Halogen, Hydroxy, primärem, sekundärem oder tertiärem Amin, gegebenenfalls substituiertem C₁₋₄-Alkyl, gegebenenfalls substituiertem C₂₋₅-Alkenyl, gegebenenfalls substituiertem C₂₋₆-Alkynyl, gegebenenfalls substituiertem Aryl, gegebenenfalls substituiertem Heteroaryl, gegebenenfalls substituiertem Heterocyclus und gegebenenfalls substituiertem C₃-C₇-Cycloalkyl;
und der andere von R⁶ und R⁷ ist A-Y-Z, worin
-A- ausgewählt Ist aus der Gruppe bestehend aus Hydrogen, -C(R²)(R²)-, -C(=O)-, -N(R^{N})-, -O-, -S-, -P-, -P(O)-;
Y ist ein Biradikal, das eventuell abwesend ist oder unabhängig ausgewählt ist aus einer der Gruppen bestehend aus gegebenenfalls substituiertem C₁₋₆-Alkyl, gegebenenfalls substituiertem C(=O)-C₁₋₆₋Alkyl, gegebenenfalls substituiertem C₁₋₆-Alkyl-C(=O), gegebenenfalls substituiertem C₂₋₁₀-Alkenyl, gegebenenfalls substituiertem C₂₋₁₀-Alkynyl, gegebenenfalls substituiertem C₃₋₈-Carbocyclus und gegebenenfalls substituiertem Heterocyclus;
Z ist ein Monoradikal, das unabhängig ausgewählt ist aus der Gruppe bestehend aus gegebenenfalls substituiertem Aryl, gegebenenfalls substituiertem Heteroaryl, gegebenenfalls substituiertem C₃₋₈-Carbocylus, gegebenenfalls substituiertem Heterocyclus, H, OR^{Z}, N(R^{Z})(R³), S-R^{Z}, NO₂, -CN und Halogen;
wo R^{Z} ausgewählt ist aus der Gruppe bestehend aus Hydrogen, gegebenenfalls substituiertem C₁₋₄-Alkyl, gegebenenfalls substituiertem C₂₋₅-Alkenyl, gegebenenfalls substituiertem C₂₋₆-Alkynyl, gegebenenfalls substituiertem Aryl, gegebenenfalls substituiertem Heteroaryl, gegebenenfalls substituiertem Heterocyclus und gegebenenfalls substituiertem C₃-C₇-Cycloalkyl;
der Ausdruck "gegebenenfalls substituiertem" bedeutet, dass die betreffende Gruppe eventuell 1 bis 5 Mal substituiert ist mit einer oder mehreren Gruppe(n), die ausgewählt ist/sind aus C₁₋₆-Alkyl, C₁₋₆-Alkoxyl, Oxo (eventuell in der tautomerlschen Enolform dargestellt), Carboxyl, Amino, Hydroxyl (das, wenn es in einem Enolsystem vorhanden ist, eventuell in der tautomerischen Ketoform dargestellt ist), Nitro, Sulphono, Sulphanyl, Sulfoxid, C₁₋₆-Carboxyl, C₁₋₆-Alkoxycarbonyl, C₁₋₆-Alkylcarbonyl, Formyl, Aryl, Aryloxy, Aryloxycarbonyl, Arylcarbonyl, Heteroaryl, Amino, Mono- und Di (C₁₋₆-Alkyl)amino; Carbamoyl, Mono- und Di(C₁₋₆-Alkyl)aminocarbonyl, Amino-C₁₋₆-Alkyl-Aminocarbonyl, Mono- und Di(C₁₋₆-Alkyl)amino-C₁₋₆-Alkyl-Aminocarbonyl, C₁₋₆-Alkylcarbonylamino, Cyano, Guanidino, Carbamido, C₁₋₆-Alkanoyloxy, C₁₋₆-Alkylsulphonyloxy, Dihalogen-C₁₋₆-Alkyl, Trihalogen-C₁₋₆-Alkyl und Halogen.

2. Die Anwendung gemäß Anspruch 1, worin O^{E} und R¹ gleich =O sind.

3. Die Anwendung gemäß irgendeinem der vorhergehenden Ansprüche, worin A ausgewählt ist aus der Gruppe bestehend aus C(R²R³), N(R²), O und S, vorzugsweise N(R²), O und S, äußerst bevorzugt N(R²) und S.

4. Die Anwendung gemäß irgendeinem der vorhergehenden Ansprüche, worin Y ausgewählt ist aus der Gruppe bestehend aus C₁₋₆-Alkyl, C₂₋₁₀-Alkenyl, C₂₋₁₀-Alkynyl, von denen jedes gegebenenfalls substituiert ist, vorzugsweise worin Y gegebenenfalls mit C₁₋₆-Alkyl substituiert ist.

5. Die Anwendung gemäß irgendeinem der vorhergehenden Ansprüche, wo Z ausgewählt ist aus der Gruppe bestehend aus Hydrogen, gegebenenfalls substituiertem Aryl, gegebenenfalls substituiertem Heteroaryl, gegebenenfalls substituiertem C₃₋₈-Carbocylus, gegebenenfalls substituiertem Heterocyclus, OR^{Z}, N(R^{Z})(R³), S-R^{Z}, vorzugsweise wo Z ausgewählt ist aus der Gruppe bestehend aus Hydrogen, gegebenenfalls substituiertem Heteroaryl, gegebenenfalls substituiertem Heterocydus und N(R^{Z})(R³), äußerst bevorzugt ist Z ausgewählt aus der Gruppe bestehend aus Hydrogen, gegebenenfalls substituiertem Heterocyclus und N(R^{Z})(R³).

6. Die Anwendung gemäß irgendeinem der vorhergehenden Ansprüche, worin entweder R⁶ und R⁷ Hydrogen ist, und der andere entweder R⁶ und R⁷ A-Y-Z ist, worin Z ausgewählt ist aus der Gruppe bestehend aus N(R⁸)(R⁹) und gegebenenfalls substituiertem Heteroaryl.

7. Die Anwendung gemäß irgendeinem der Ansprüche 5 bis 6, wo das gegebenenfalls substituierte Heterocyclus ausgewählt ist aus der Gruppe bestehend aus Succinimid, Imidazol, Pyrazol, Pyrrol, Oxazol, Furazan, Barbitursäure, Thiobarbitursäure, Dioxopiperazin, Hydantoin, Dihydrouracil und 3-Alkoxyisoxazol, von denen jeder gegebenenfalls substituiert ist, vorzugsweise wo das Heterocyclus ausgewählt ist aus der Gruppe bestehend aus Succinimid, Imidazol, Pyrazol, Pyrrol, Oxazol und Furazan, von denen jeder gegebenenfalls substituiert ist.

8. Die Anwendung gemäß irgendeinem der Ansprüche 3 bis 7, wo R¹ =O ist, und mindestens ein von R⁶ und R⁷ Hydrogen sind.

9. Die Anwendung gemäß Anspruch 1, wo R⁶ und R⁷ zusammen einen Ring bilden können, der ausgewählt ist aus der Gruppe bestehend aus C₃₋₈-Carbocyclus, Heterocyclus, Aryl oder Heteroaryl, von denen jedes gegebenenfalls substituiert ist.

10. Die Anwendung gemäß Anspruch 8, wo R⁶ und R⁷ zusammen ein C₃₋₈-Carbocyclus bilden können, zum Beispiel Cyclohexan.

11. Die Anwendung gemäß Anspruch 8, wo O^{E} =O ist, R¹ =O ist, R^{N} Hydrogen ist, ein von R⁶ und R⁷ Hydrogen sind, und der andere von R⁶ und R⁷ C₁₋₆-Alkyl sind.

12. Die Anwendung gemäß Anspruch 1, wo ein von R⁶ und R⁷ Hydrogen sind, und der andere von R⁶ und R⁷ ausgewählt sind aus Methyl oder Ethyl.

13. Die Anwendung gemäß Anspruch 1, wo die Verbindung der Formel M die Verbindung TT0043 ist, wo O^{E} =O ist, R¹ =O ist, R^{N} Hydrogen ist, ein von R⁶ und R⁷ Hydrogen sind, und der andere von R⁶ und R⁷ Methyl sind.

14. Die Anwendung gemäß Anspruch 1 bis 2, wo ein von R⁶ und R⁷ Hydrogen sind, und der andere von R⁶ und R⁷ A-Y-Z sind, wo A ausgewählt ist aus der Gruppe bestehend aus Hydrogen, -C(R²)(R²)-, -C(=O)-, -N(R^{N})-, -O-, -S-, -P-, -P(O)-; Y ist eventuell abwesend oder ausgewählt aus einer der Gruppen bestehend aus gegebenenfalls substituiertem C₁₋₆₋Alkyl, gegebenenfalls substituiertem C(=O)-C₁₋₆-Alkyl, gegebenenfalls substituiertem C₁₋₆₋Alkyl-C(=O), gegebenenfalls substituiertem C₂₋₁₀-Alkenyl, gegebenenfalls substituiertem C₂₋₁₀-Alkynyl, gegebenenfalls substituiertem C₃₋₈-Carbocyclus und gegebenenfalls substituiertem Heterocyclus; und Z ist ein Monoradikal, das ausgewählt ist aus der Gruppe bestehend aus gegebenenfalls substituiertem Aryl, gegebenenfalls substituiertem Heteroaryl, gegebenenfalls substituiertem C₃₋₈-Carbocylus, gegebenenfalls substituiertem Heterocyclus, H, OR^{Z}, N(R^{Z})(R³), S-R^{Z}, NO₂, -CN und Halogen.

15. Die Anwendung gemäß Anspruch 14, wo ein von R⁶ und R⁷ Hydrogen sind, und der andere von R⁶ und R⁷ A-Y-Z sind, wo A ausgewählt ist aus der Gruppe bestehend aus Hydrogen, -C(R²)(R²)-, -C(=O)-, -N(R^{N})-, -O-, -S-, -P-, -P(O)-; Y ist eventuell abwesend oder ausgewählt aus einer der Gruppen bestehend aus gegebenenfalls substituiertem C₁₋₆-Alkyl, gegebenenfalls substituiertem C(=O)-C₁₋₆-Alkyl, gegebenenfalls substituiertem C₁₋₆-Alkyl-C(=O), gegebenenfalls substituiertem C₂₋₁₀-Alkenyl, gegebenenfalls substituiertem C₂₋₁₀-Alkynyl, gegebenenfalls substituiertem C₃₋₆-Carbocyclus und gegebenenfalls substituiertem Heterocyclus; und wo Z ausgewählt ist aus der Gruppe bestehend aus N(R⁸)(R⁹), gegebenenfalls substituiertem Heteroaryl und gegebenenfalls substituiertem Heterocyclus.

16. Die Anwendung gemäß Anspruch 15, wo Z N(R⁸)(R⁹) ist, wo R⁸ und R⁹ eventuell unabhängig ausgewählt sind aus der Gruppe bestehend aus Hydrogen, gegebenenfalls substituiertem C₁₋₆-Alkyl, gegebenenfalls substituiertem C₂₋₁₀-Alkenyl, gegebenenfalls substituiertem C₂₋₁₀-Alkynyl, gegebenenfalls substituiertem Aryl, gegebenenfalls substituiertem Heteroaryl, gegebenenfalls substituiertem Heterocyclus, gegebenenfalls substituiertem C₃-C₇-Cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂-S-C(=O)R³, C(=O)(R³), C(=S)R³, -C(=S)-OR³, -C(=O)-SR³, C(=O)-N(R³)(R³) und C(C=S)-N(R³)(R³), vorzugsweise wo mindestens ein von R⁸ und R⁹ ausgewählt sind aus der Gruppe bestehend aus gegebenenfalls substituiertem Heteroaryl und gegebenenfalls substituiertem Heterocyclus, äußerst bevorzugt gegebenenfalls substituiertem Heterocyclus.

17. Die Anwendung gemäß Anspruch 1, wo die Verbindung der Formel M ausgewählt Ist aus der Gruppe bestehend aus Maleimid, N-Methylmaleimid (NMM) und N-Ethylmaleimid (NEM).

18. Die Anwendung gemäß Anspruch 1, wo die Verbindung der Formel M die Verbindung 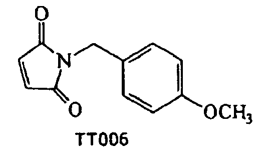
TT006 ist,

19. Die Anwendung gemäß Anspruch 1, wo die Verbindung der Formel M die Verbindung 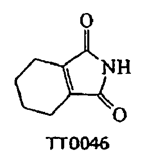
TT0046 ist,

20. Die Anwendung gemäß Anspruch 1, wo die Verbindung der Formel M die Verbindung 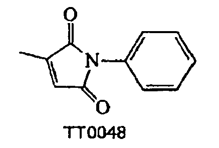
TT0048 ist,

21. Die Anwendung gemäß Anspruch 1, wo die Verbindung der Formel M die Verbindung 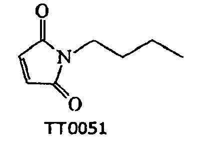
TT0051 ist,

22. Die Anwendung gemäß Anspruch 1, wo die Verbindung der Formel M eine Verbindung ist, die ausgewählt ist aus der Gruppe bestehend aus M-i, M-ii, M-iii, M-iv, M-v, M-vi und M- 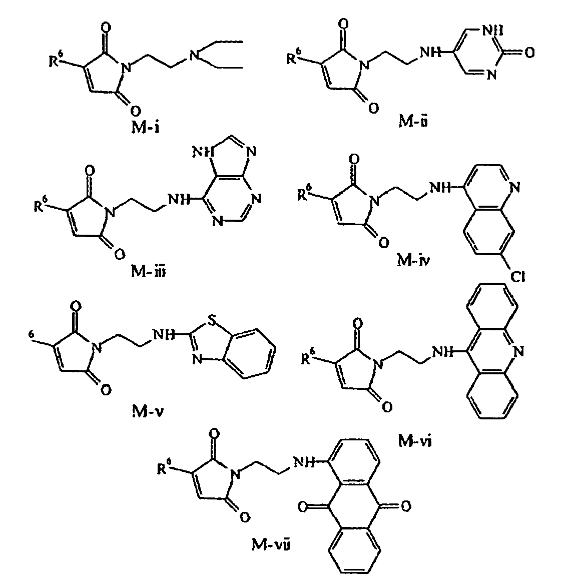
vii,
wo R⁶ A-Y-Z ist, wo A ausgewählt ist aus der Gruppe bestehend aus Hydrogen, -C(R²)(R²)-, -C(=O)-, -N(R^{N})-, -O-, -S-, -P-, -P(O)-; Y ist eventuell abwesend oder ausgewählt ist aus einer der Gruppen bestehend aus gegebenenfalls substituiertem C₁₋₆-Alkyl, gegebenenfalls substituiertem C(=O)-C₁₋₆-Alkyl, gegebenenfalls substituiertem C₁₋₆-Alkyl-C(=O), gegebenenfalls substituiertem C₂₋₁₀Alkenyl, gegebenenfalls substituiertem C₂₋₁₀-Alkynyl, gegebenenfalls substituiertem C₃₋₈-Carbocyclus und gegebenenfalls substituiertem Heterocydus; und Z ist ein Monoradikal, das ausgewählt ist aus der Gruppe bestehend aus gegebenenfalls substituiertem Aryl, gegebenenfalls substituiertem Heteroaryl, gegebenenfalls substituiertem C₃₋₈-Carbocylus, gegebenenfalls substituiertem Heterocyclus, H, OR^{Z}, N(R^{Z})(R³), S-R^{Z}, NO₂, -CN und Halogen.

23. Die Anwendung gemäß Anspruch 1, wo die Verbindung der Formel M eine Verbindung ist, die ausgewählt ist aus der Gruppe bestehend aus M-vill, M-ix, M-x und M-xi, 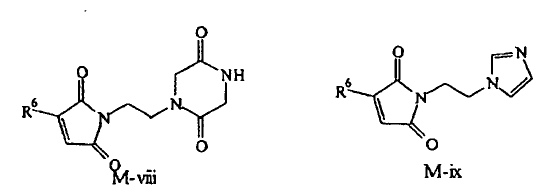 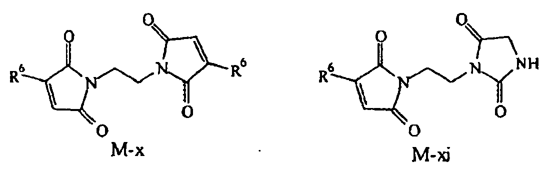
wo R⁶ A-Y-Z ist, wo A ausgewählt ist aus der Gruppe bestehend aus Hydrogen, -C(R²)(R²)-, -C(=O)-, -N(R")-, -O-, -S-, -P-, -P(O)-; Y ist eventuell abwesend oder ausgewählt aus einer der Gruppen bestehend aus gegebenenfalls substituiertem C₁₋₆-Alkyl, gegebenenfalls substituiertem C(=O)-C₁₋₆-Alkyl, gegebenenfalls substituiertem C₁₋₆-Alkyl-C(=O), gegebenenfalls substituiertem C₂₋₁₀-Alkenyl, gegebenenfalls substituiertem C₂₋₁₀-Alkynyl, gegebenenfalls substituiertem C₃₋₈-Carbocyclus und gegebenenfalls substituiertem Heterocyclus; und Z ist ein Monoradikal, das ausgewählt ist aus der Gruppe bestehend aus gegebenenfalls substituiertem Aryl, gegebenenfalls substituiertem Heteroaryl, gegebenenfalls substituiertem C₃₋₈-Carbocylus, gegebenenfalls substituiertem Heterocyclus, H, OR^{Z}, N(R^{Z})(R³), S-R^{Z}, NO₂, -CN und Halogen.

24. Die Anwendung gemäß Anspruch 1, wo die Verbindung der Formel M eine Verbindung 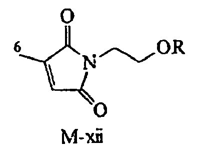
der Formel M-xii ist,
wo OR ausgewählt Ist aus einem Alkohol, einem Ether und einem Ester.

25. Die Anwendung gemäß Anspruch 1, wo die Verbindung der Formel M eine Verbindung der Formel M-xiii ist, 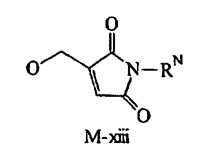
wo OR ausgewählt ist aus einem Alkohol, einem Ether und einem Ester, und
R^{N} ist ausgewählt aus der Gruppe bestehend aus Hydrogen, gegebenenfalls substituiertem C₁₋₆-Alkyl, gegebenenfalls substituiertem C₂₋₁₀-Alkenyl, gegebenenfalls substituiertem C₂₋₁₀₋Alkynyl, gegebenenfalls substituiertem Aryl, gegebenenfalls substituiertem Heteroaryl, gegebenenfalls substituiertem Heterocyclus, gegebenenfalls substituiertem C₃-C₇-Cycloalkyl, CH₂-N(R³)(R³), CH₂-OR³, CH₂-SR³, CH₂-O-C(=O)R³, CH₂-O-C(=O)-OR³, CH₂-O-C(=S)R³, CH₂₋S-C(=O)R³, C(=O)(R³), C(=S)R³, -C(=S)-OR³, -C(=O)-SR³, C(=O)-N(R³)(R³), C(C=S)-N(R³)(R³).

26. Anwendung gemäß irgendeinem der Ansprüche 1 bis 25, wo die Extravasation aufgrund von einem Topoisomerase II-Gift entsteht.

27. Anwendung einer Verbindung der Formel M wie in irgendeinem der Ansprüche 1 bis 26 definiert, quartärer Ammoniumsalze hiervon oder Zusammensetzungen unfassend irgendeine Einheit zur Herstellung eines Arzneimittels für die Behandlung von Krebs.

28. Anwendung einer Kombination von einem Topoisomerase II-Gift und einer Verbindung der Formel M wie in irgendeinem der Ansprüche 1 bis 26 definiert, quartärer Ammoniumsalze hiervon oder Zusammensetzungen unfassend irgendeine Einheit zur Herstellung eines Arzneimittels für die Behandlung von Krebs.

29. Anwendung gemäß Anspruch 28, wo das betreffende Topoisomerase II-Gift ausgewählt ist aus der Gruppe bestehend aus Doxorubicin, Daunorubicin, Dactinomycin, Epirubicin, Bisantren, Amsacrin, Mitomycin C, Vincristin, Vinblastin, Vindesin, liposomalem Anthracyclines, Mitoxantron, Esorubicin, Menogaril, Acalcinomycin, Cisplatin, Fluorouracil, Etoposid und Bleomycin.

30. Eine pharmazeutische Zusammensetzung umfassend mindestens eine Verbindung, die ausgewählt ist aus der Gruppe bestehend aus der Formel M, wie in irgendeinem der vorhergehenden Ansprüche definiert, und ein oder mehrere chemotherapeutische(s) Mittel, das/die ausgewählt ist/sind aus der Gruppe bestehend aus Doxorubicin, Daunorubicin, Dactinomycin, Epirubicin, Blsantren, Amsacrin, Mitomycin C, Vincristin, Vinblastin, Vindesin, liposomalem Anthracyclines, Mitoxantron, Esorubicin, Menogaril, Acalcinomycin, Cisplatin, Fluorouracil, Etoposid und Bleomycin, und mindestens ein pharmazeutisch akzeptables Bindemittel oder Trägermittel.

31. Eine Zusammensetzung gemäß Anspruch 30, wo die Verbindung der Formel M aus der Formel Maleimid, NMM, NEM, TT006, TT0043, TT0046, TT0048, TT0051, M-i, M-ii, M-iii, M-iv, M-v, M-vi, M-vii, M-viii, M-ix, M-x, M-xi, M-xii, M-xiii ist, wie in den Ansprüchen 13 und 17-25 definiert.
